# EUROPEAN PATENT APPLICATION

(11) **EP 4 296 282 A1**
(43) Date of publication of application: **27.12.2023**
(21) Application number: 22756590.0
(22) Date of filing: 21.02.2022
(51) Int. Cl.: C07K 16/28, A61P 35/00, A61K 39/00

(54) **SINGLE DOMAIN ANTIBODY AGAINST CD47 AND USE THEREOF**

(30) Priority: 19.02.2021 KR 20210022805
(71) Applicant: SHAPERON Inc., Seoul 06373 (KR); Seoul National University R & DB Foundation, Seoul 08826 (KR)
(72) Inventor: SEONG, Seung Yong, Seoul 05507 (KR); LEE, Sang Beum, Seoul 06374 (KR)
(74) Representative: Vossius & Partner Patentanwälte Rechtsanwälte mbB
(86) International application number: PCT/KR2022/002527
(87) International publication number: WO 2022/177392

(57) **Abstract**

The present invention relates to a single domain antibody against CD47 and uses thereof. Specifically, a single domain antibody that specifically binds to CD47, an immune checkpoint protein, has been constructed and its affinity for an immune antigen and antitumor effect have been verified, so the single domain antibody can be usefully used as an immune checkpoint inhibitor in cancer immunotherapy.

## Description

### [Technical Field]

The present invention relates to a single domain antibody against CD47, an immune checkpoint protein, and uses thereof.

### [Background Art]

Recently, as the therapeutic effect of newly developed cancer immunotherapy using the immune system of human body has been proven, anticancer therapy using conventional chemotherapeutic agents and targeted cancer drugs is changing to cancer immunotherapy using immunotherapeutic agents.

Basically, the immune cells of cancer patients have developed tolerance to cancer antigens to be able to recognize cancer cells, but are functionally suppressed, being unavailable effectively eliminate cancer cells. The core of immunotherapy is to stimulate immune cells having tolerance and induce the activated immune cells to destroy cancer cells. Such immunotherapy includes cytokines, such as IFN-γ and IL-2, as therapeutic agents, cancer vaccines using dendritic cells, cell therapy products using T cells, and immune checkpoint inhibitors (ICIs) that block immune checkpoint proteins, and these therapeutic agents are commonly called immuno-oncology therapies. Among these, immuno-oncology drugs that international pharmaceutical companies are competitively developing are immune checkpoint inhibitors.

Immune checkpoint proteins are cell membrane proteins that inhibit the differentiation, proliferation, and activity of immune cells. Specifically, the proteins are mostly expressed in activated T cells, have functions to reduce T cell proliferation, cytokine secretion, and cytotoxicity and inhibit excessive T cell activity, and so are also called co-inhibitory molecules. Representatively, CTLA-4 and PD-1, which are co-inhibitory receptors, are expressed in T cells and bind to B7.1/2 and PD-L1, respective ligands, to regulate T cell activity. Meanwhile, PD-L1 is expressed in cancer cells, inactivates cancer-specific T cells, induces apoptosis, plays a role as an important molecular shield that protects cancer cells from immune attack by T cells, and thus serves as a cancer immune evasion mechanism. It has been reported that cancer patients in which PD-L1 is expressed ectopically in cancer cells have a worse prognosis than cancer patients in which PD-L1 is not expressed.

Immune checkpoint inhibitors are drugs that block the activity of immune checkpoint proteins involved in suppressing these T cells to activate T cells, thereby attacking cancer cells, and representatively use antibodies that recognize CTLA-4, PD-1, and PD-L1. Ipilimumab (Yervoy), a CTLA-4 inhibitor, has first received FDA approval in 2011 as a second-line therapeutic agent for metastatic melanoma among immune checkpoint inhibitors. Thereafter, in 2014, nivolumab (Opdivo) and pembrolizumab (Keytruda), PD-1 blockers, have each received FDA approval for metastatic melanoma. Thereafter, atezolizumab (Tecentriq), an immune checkpoint inhibitor of PD-L1, has received FDA approval for bladder cancer (2016), averumab (Bavencio) has received FDA approval for the treatment of metastatic merkel cell carcinoma, which is a type of skin cancer, and durvalumab (Imfinzi) has received FDA approval in 2017 for bladder cancer. In 2018, chemiplimab (Libtayo), a PD-1 inhibitor, has received FDA approval for squamous cell carcinoma of the skin. Currently, these drugs have expanded their therapeutic indications and received FDA approval for the treatment of a growing number of carcinomas. As of 2019, six PD-1/PD-L1 inhibitors have received FDA approval for a total of 18 carcinomas. In addition, immunomodulatory proteins such as B7-H4, ICOS, HVEM, PDL-2, and PVRIG are entering preclinical trials as new targets. Most of these therapeutic agents target targets expressed on T cells.

In order to overcome the biased target discovery tendency centered on T cells, inhibitors targeting immune checkpoint proteins expressed in myeloid cells, such as macrophages and dendritic cells, have recently been developed. Among these, CSF1R, CD47, and TLR7 are emerging as important targets.

CD47 (Cluster of Differentiation 47), which was first identified as a tumor antigen in human ovarian cancer in 1980, is expressed in a number of human tumor cells, including non-Hodgkin's lymphoma (NHL), multiple myeloma (MM), acute lymphoblastic leukemia (ALL), acute myelogenous leukemia (AML), chronic myelogenous leukemia, bladder cancer and solid cancers.

CD47 is expressed on the cell surface and interacts with SIRP alpha (SIRPa), thrombospondin-1 (TSP1) and integrin proteins to participate in cell death, proliferation and immune response. In particular, CD47 expressed in tumor cells interacts with SIRP alpha (SIRPa) expressed on the surface of macrophages to send a "don't eat me" signal, thereby avoiding phagocytosis of macrophages as well as inhibits vascular proliferation and the role of effector T-cells in the tumor environment to promote proliferation and growth of tumor cells.

Magrolimab of Gilead Sciences Inc., a global pharmaceutical company, is undergoing clinical trials as an antibody therapeutic agent targeting CD47, and multinational global pharmaceutical companies have recently actively developed bispecific antibodies based on CD47 antibody therapeutic agents.

Accordingly, the present inventors have developed an immune checkpoint inhibitor targeting an immune checkpoint protein, leading to the present application.

### [Citation List]

### [Patent Literature]

[Patent Literature 1]
   US Patent Publication No. 2020-0181259
[Patent Literature 2]
   International Patent Publication No. 2011-143624

### [Non Patent Literature]

[Non Patent Literature 1]
   Pol Specenier (2016): Ipilimumab in melanoma, Expert Review of Anticancer Therapy.
[Non Patent Literature 2]
   DB Johnson, C Peng et al. Nivolumab in melanoma: latest evidence and clinical potential. Ther Adv Med Oncol 2015, Vol. 7(2) 97-106
[Non Patent Literature 3]
   Liu J, Wang L, Zhao F, Tseng S, Narayanan C, Shura L, et al. (2015) Pre-Clinical Development of a Humanized Anti-CD47 Antibody with Anti-Cancer Therapeutic Potential. PLoS ONE 10(9): e0137345.

### [Summary of Invention]

### [Technical Problem]

An object of the present invention is to provide a single domain antibody (sdAb) against CD47, an immune checkpoint protein, and uses thereof.

### [Solution to Problem]

In order to achieve the object of the present invention, the present invention provides an antibody comprising a single domain antibody (sdAb) that specifically binds to CD47 or an antigen-binding fragment thereof, in which
the sdAb includes CDR1 consisting of an amino acid sequence represented by any one of SEQ ID NOs: 3 and 6; CDR2 consisting of an amino acid sequence represented by any one of SEQ ID NOs: 4 and 7; and CDR3 consisting of an amino acid sequence represented by any one of SEQ ID NOs: 5 and 8.

In an aspect of the present invention, the sdAb may include (1) CDR1 consisting of an amino acid sequence represented by SEQ ID NO: 3; CDR2 consisting of an amino acid sequence represented by SEQ ID NO: 4; and CDR3 consisting of an amino acid sequence represented by SEQ ID NO: 5; or (2) CDR1 consisting of an amino acid sequence represented by SEQ ID NO: 6; CDR2 consisting of an amino acid sequence represented by SEQ ID NO: 7; and CDR3 consisting of an amino acid sequence represented by SEQ ID NO: 8.

The sdAb may include a VHH domain including FR1 consisting of an amino acid sequence represented by any one of SEQ ID NOs: 11 and 15; FR2 consisting of an amino acid sequence represented by any one of SEQ ID NOs: 12 and 16; FR3 consisting of an amino acid sequence represented by any one of SEQ ID NOs: 13 and 17; and FR4 consisting of an amino acid sequence represented by any one of SEQ ID NOs: 14 and 18, more specifically may include a VHH domain including (1) FR1 consisting of an amino acid sequence represented by SEQ ID NO: 11; FR2 consisting of an amino acid sequence represented by SEQ ID NO: 12; FR3 consisting of an amino acid sequence represented by SEQ ID NO: 13; and FR4 consisting of an amino acid sequence represented by SEQ ID NO: 14; or (2) FR1 consisting of an amino acid sequence represented by SEQ ID NO: 15; FR2 consisting of an amino acid sequence represented by SEQ ID NO: 16; FR3 consisting of an amino acid sequence represented by SEQ ID NO: 17; and FR4 consisting of an amino acid sequence represented by SEQ ID NO: 18, and in some embodiments, includes an amino acid sequence represented by any one of SEQ ID NOs: 1 and 2.

In an aspect of the present invention, the sdAb may contain at least one or more amino acid substitutions, and the at least one or more amino acid substitutions may be conservative substitutions, and may be a substitution of an amino acid with a non-genetically encoded amino acid or a synthetic amino acid.

In an aspect of the present invention, the sdAb may be monomeric or multimeric. In a case of being multimeric, the sdAbs may be fused to each other via a peptide linker.

In an aspect of the present invention, a heavy chain-only antibody (HCAb) in which the sdAb is fused to an Fc fragment is provided. In some embodiments, the HCAb may consist of an amino acid sequence represented by any one of SEQ ID NOs: 9, 10 and 19.

In an aspect of the present invention, the HCAb may be monomeric or multimeric.

In an aspect of the present invention, an sdAb may be fused to the Fc fragment via a peptide linker, and the Fc fragment may be human IgG1, IgG2, IgG3 or IgG4.

In an aspect of the present invention, the HCAb may contain at least one or more amino acid substitutions, and the at least one or more amino acid substitutions may be conservative substitutions, and may be a substitution of an amino acid with a non-genetically encoded amino acid or a synthetic amino acid.

In an aspect of the present invention, an antibody comprising (a) a first antigen-binding moiety including the sdAb; and (b) a second antigen-binding moiety that specifically binds to a second epitope is provided.

In an aspect of the present invention, the antibody may be bispecific or multispecific.

In an aspect of the present invention, the second antigen-binding moiety may be fused to the first antigen-binding moiety via a peptide linker, and the second antigen-binding moiety may be a full-length antibody, Fab, Fab', (Fab')2, Fv, single chain Fv (scFv), scFv-scFv, minibody, diabody or second sdAb.

In an aspect of the present invention, an immunomodulator, cytokine, cytotoxic agent, chemotherapeutic agent, diagnostic agent, antiviral agent, antimicrobial agent or drug may be conjugated. Accordingly, the present invention provides an antibody conjugate comprising the antibody or an antigen-binding fragment thereof, which is conjugated to an immunomodulator, cytokine, cytotoxic agent, chemotherapeutic agent, diagnostic agent, antiviral agent, antimicrobial agent or drug.

The present invention also provides a nucleic acid molecule encoding the antibody or an antigen-binding fragment thereof.

The present invention also provides an expression vector comprising the nucleic acid molecule.

The present invention also provides a host cell transformed with the expression vector.

The present invention also provides a method for producing an antibody or an antigen-binding fragment thereof, which comprises:
(a) culturing the host cell under conditions that allow expression of the antibody; and
(b) recovering the expressed antibody or an antigen-binding fragment thereof.

The present invention also provides a pharmaceutical composition for prevention or treatment of cancer, containing the antibody or an antigen-binding fragment thereof or the antibody conjugate as an active ingredient; a method for preventing or treating cancer, which comprises administering the antibody or an antigen-binding fragment thereof or the antibody conjugate to an individual in a pharmaceutically effective amount; and a use of the antibody or an antigen-binding fragment thereof or the antibody conjugate for prevention or treatment of cancer.

In an aspect of the present invention, the cancer may be selected from the group consisting of melanoma, lung cancer, liver cancer, glioblastoma, ovarian cancer, colorectal cancer, head and neck cancer, bladder cancer, renal cell cancer, stomach cancer, breast cancer, metastatic cancer, prostate cancer, pancreatic cancer, non-Hodgkin's lymphoma, Hodgkin's lymphoma, multiple myeloma, leukemia, lymphoma, myelodysplastic syndrome, acute lymphoblastic leukemia, acute myelogenous leukemia, chronic lymphocytic leukemia, chronic myelogenous leukemia, solitary myeloma and aplastic anemia.

In an aspect of the present invention, the pharmaceutical composition may further contain a pharmaceutically acceptable carrier.

The present invention also provides a method for detecting CD47 or determining an amount of CD47 in a sample, which comprises:
(i) bringing the sample into contact with the antibody or an antigen-binding fragment; and
(ii) detecting formation of a complex between the antibody or an antigen-binding fragment thereof and CD47 or determining an amount of the complex.

### [Advantageous Effects of Invention]

In the present invention, a single domain antibody that specifically binds to CD47, an immune checkpoint protein, has been constructed and its affinity for an immune antigen and antitumor effect have been verified, so the single domain antibody can be usefully used as an immune checkpoint inhibitor in cancer immunotherapy.

### [Brief Description of Drawings]

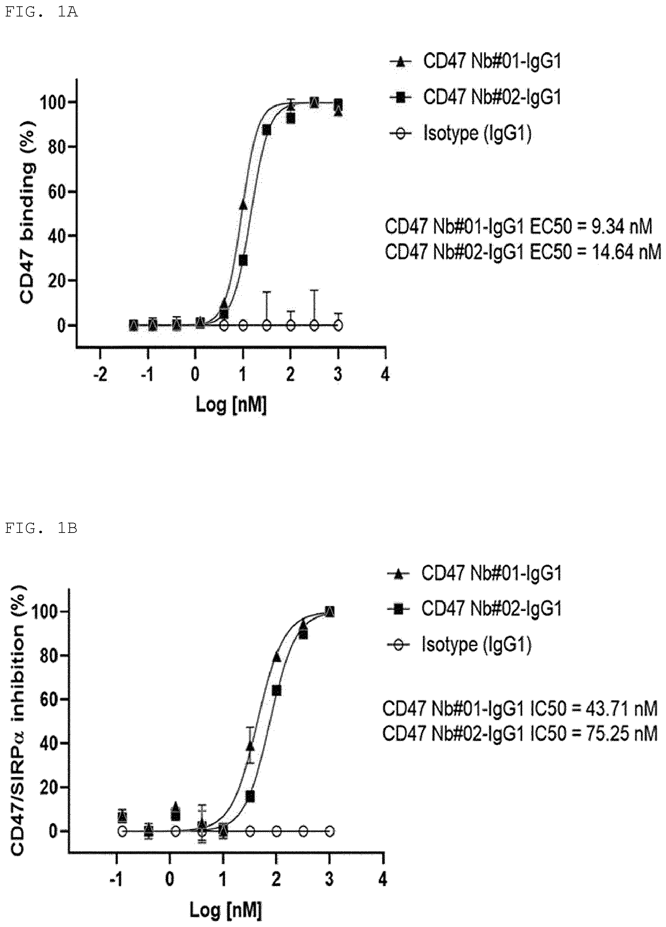

FIG. 1A is a diagram confirming the binding capacity (EC50) to a CD47 antigen expressed on the cell surface by reacting an Expi-CHO_CD47 cell line (Expi-CHO cells induced to express the CD47 antigen) and anti-CD47 HCAbs (CD47 Nb#01-IgG1 and CD47 Nb#02-IgG1) constructed according to Example of the present invention at each concentration;
FIG. 1B is a diagram confirming the inhibitory ability (IC50) of anti-CD47 HCAbs (CD47 Nb#01-IgG1 and CD47 Nb#02-IgG1) on the CD47/SIRPalpha interaction after binding of an Expi-CHO_CD47 cell line (Expi-CHO cells induced to express the CD47 antigen) constructed according to Example of the present invention to a SIRPalpha protein;

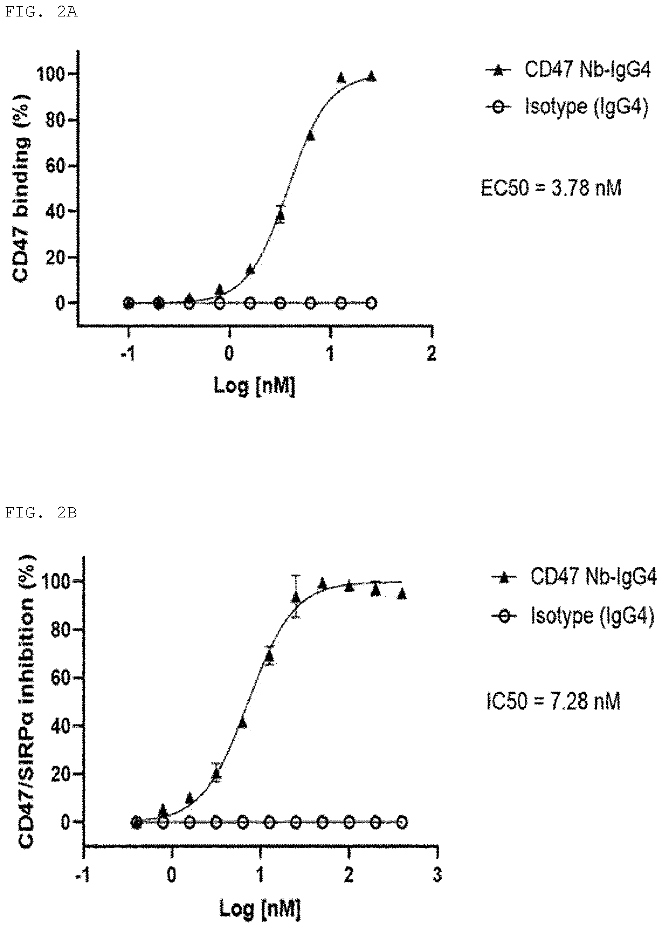

FIG. 2A is a diagram confirming the binding capacity (EC50) to a CD47 antigen expressed on the cell surface by reacting an Expi-CHO_CD47 cell line (Expi-CHO cells induced to express the CD47 antigen) and an anti-CD47 HCAb (CD47 Nb-IgG4) constructed according to Example of the present invention at each concentration;
FIG. 2B is a diagram confirming the inhibitory ability (IC50) of an anti-CD47 HCAb (CD47 Nb-IgG4) on the CD47/SIRPalpha interaction after binding of an Expi-CHO_CD47 cell line (Expi-CHO cells induced to express the CD47 antigen) constructed according to Example of the present invention to a SIRPalpha protein;

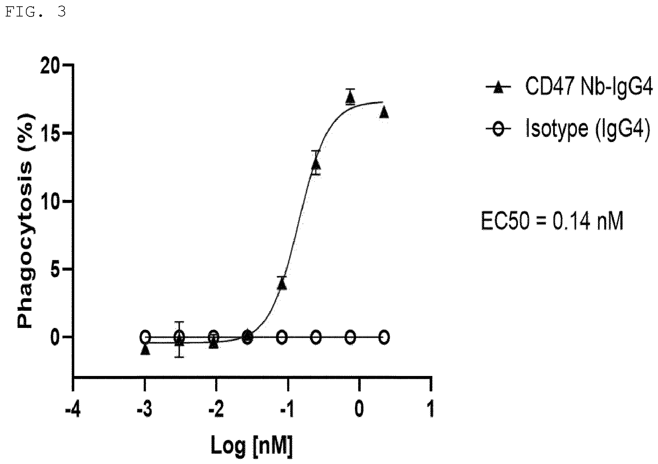

FIG. 3 is a diagram confirming phagocytosis of an anti-CD47 HCAb (CD47 Nb-IgG4) constructed according to Example of the present invention;

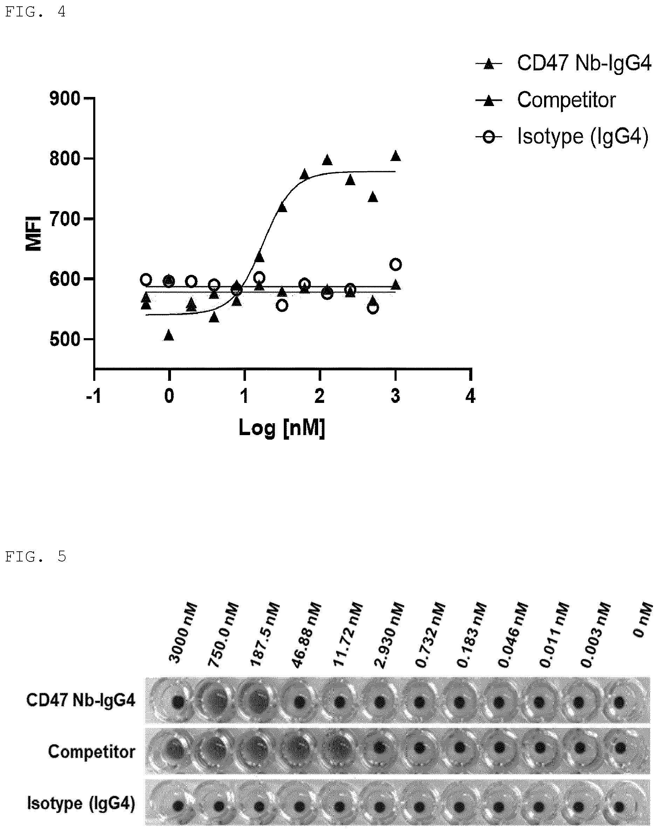

FIG. 4 is a diagram confirming the human RBC binding capacity of an anti-CD47 HCAb (CD47 Nb-IgG4) constructed according to Example of the present invention;
FIG. 5 is a diagram confirming human hemagglutination of an anti-CD47 HCAb (CD47 Nb-IgG4) constructed according to Example of the present invention; and

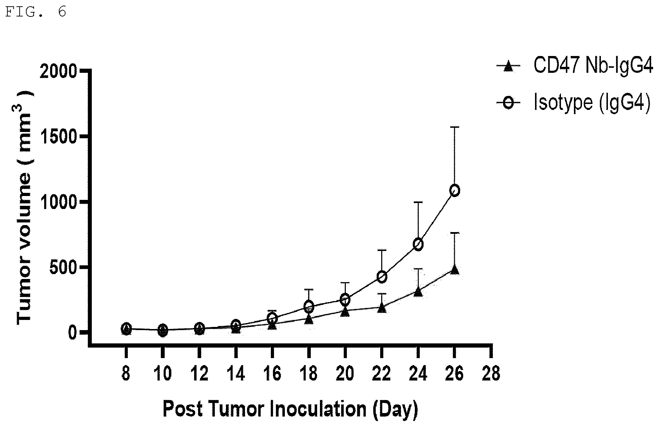

FIG. 6 is a diagram confirming the antitumor effect of an anti-CD47 HCAb (CD47 Nb-IgG4) constructed according to Example of the present invention after intraperitoneal administration of the anti-CD47 HCAb (CD47 Nb-IgG4) to C57BL/6 mice in which tumor formation is induced with a B16F10_CD47 cell line (a tumor cell line induced to express the CD47 antigen).

### [Description of Embodiments]

Hereinafter, embodiments of the present invention will be described in detail so that those skilled in the art to which the present invention pertains can easily implement the present invention. Embodiments of the present invention are provided to more completely describe the present invention to those with average knowledge in the art. Therefore, embodiments of the present invention may be modified in a number of different forms, and the scope of the present invention is not limited to the embodiments described below.

In the present invention, the term "epitope" refers to a protein determinant capable of specifically binding to an antibody. Epitopes generally consist of chemically active surface groupings of molecules, for example, amino acids or sugar side chains, and generally have specific three-dimensional structural characteristics as well as specific charge characteristics.

The term "treatment" may refer to slowing, stopping, halting, controlling, bring to a standstill, alleviating or ameliorating a disorder or disease disclosed herein, for example, a symptom or complication of a disease, or reversing its progression, and refers to any process that does not necessarily represent complete elimination of all symptoms of a disease or disorder.

The term "prevention" refers to prophylactic treatment of a disease or disorder, for example, a disease, or delaying the onset or progression of a disease or disorder.

The term "individual" or "subject" refers to, but not limited to, a mammal including a human, bovine, equine, feline, canine, rodent or a primate. In some embodiments, the individual is a human.

The term "antibody" is used in its broadest sense and encompasses a variety of antibody structures, including but not limited to monoclonal antibodies, polyclonal antibodies, multispecific antibodies (for example, bispecific antibodies), full-length antibodies, and antigen-binding fragments thereof, as long as they exhibit the desired antigen-binding activity. The term "antibody" includes conventional four-chain antibodies, single domain antibodies, and antigen-binding fragments thereof.

A basic four-chain antibody unit is a heterotetrameric glycoprotein composed of two identical light (L) chains and two identical heavy (H) chains. IgM antibodies consist of five of the basic heterotetrameric units together with an additional polypeptide called the J chain and contain ten antigen-binding sites, while IgA antibodies contain two to five of the basic four-chain units that can polymerize to form multivalent assemblies in combination with the J chain. In the case of IgG, the four-chain unit is generally about 150,000 daltons. Each L chain is linked to the H chain by one covalent disulfide bond, while two H chains are linked to each other by one or more disulfide bonds depending on the H chain isotype. Each H chain and each L chain also have regularly spaced interchain disulfide bridges. Each H chain has, at its N-terminus, a variable domain (VH) followed by three constant domains (CH) for each of the α and γ chains and four CH domains for the µ and ε isotypes. Each L chain has, at its N-terminus, at its other end, a variable domain (VL) followed by a constant domain. VL aligns with VH and CL aligns with the first constant domain (CH1) of the heavy chain. Pairing of VH and VL forms a single antigen-binding site. L chains from any vertebrate species may be assigned to one of two clearly distinct types, called kappa and lambda, based on the amino acid sequences of their constant domains. Depending on the amino acid sequences of the constant domains (CH) of their heavy chains, immunoglobulins may be assigned to different classes or isotypes. There are five classes of immunoglobulins: α, δ, ε, γ and µ, each with a designated heavy chain, IgA, IgD, IgE, IgG and IgM. The γ and α classes are further divided into subclasses based on relatively few differences in CH sequence and function; for example, humans express the following subclasses: IgG1, IgG2A, IgG2B, IgG3, IgG4, IgA1 and IgA2.

The term "heavy chain-only antibody" or "HCAb" refers to a functional antibody comprising a heavy chain but lacking the light chain normally found in four-chain antibodies.

The term "single-domain antibody", "nanobody" or "sdAb" refers to a single antigen-binding polypeptide having three complementarity determining regions (CDRs). An sdAb alone can bind to an antigen without pairing with the corresponding CDR-containing polypeptide. In some cases, single-domain antibodies are engineered from Camelid HCAbs, and their heavy chain variable domains are referred to herein as "VHH" (variable domain of the heavy chain of heavy chain antibodies). A basic VHH has the following structure from N-terminus to C-terminus: FR1-CDR1-FR2-CDR2-FR3-CDR3-FR4, where FR1 to FR4 refer to framework regions 1 to 4, respectively, and CDR1 to CDR3 refer to complementarity determining regions 1 to 3.

A "variable region" or "variable domain" of an antibody refers to the amino-terminal domain of the heavy or light chain of the antibody. The variable domains of heavy and light chains may be referred to as "VH" and "VL", respectively. These domains are generally the most variable moieties of the antibody (relative to other antibodies of the same class) and contain an antigen-binding site. Heavy chain-only antibodies from species of Camelidae have a single heavy chain variable region referred to as "VHH".

The term "variable" refers to the fact that certain segments of variable domains differ widely in sequence among antibodies. A V domain mediates antigen binding and defines the specificity of a particular antibody for its particular antigen. However, the variability is not evenly distributed over the full extent of a variable domain. Instead, variability is enriched in three segments called complementarity determining regions (CDRs) or hypervariable regions (HVRs) in both heavy and light chain variable domains. The more highly conserved portions of variable domains are called framework regions (FR). The variable domains of each of the natural heavy and light chains include four FR regions, which predominantly adopt a beta-sheet configuration, and in some cases form part of the beta-sheet structure, connected by three CDRs, forming loop connections. The CDRs on each chain are held together in close proximity by the FR regions, and the CDRs from the other chain contribute to the formation of the antigen-binding site of the antibody (see Kabat, Elvin A., Sequence of Immunological Interest, Fifth Edition, National Institute of Health, Bethesda, Md. (1991)). The constant domains are not directly involved in the binding of the antibody to its antigen, but represent participation of the antibody in various effector functions, for example, antibody-dependent cellular cytotoxicity.

The term "constant domain" refers to a part of an immunoglobulin molecule that has a more conserved amino acid sequence compared to the other part of the immunoglobulin, the variable domain, that contains the antigen-binding site. The constant domains contain the CH1, CH2 and CH3 domains (collectively, CH) of the heavy chain and the CHL (or CL) domain of the light chain.

The terms "full-length antibody", "intact antibody", or "whole antibody" are used interchangeably to refer to an antibody in its substantially intact form, as opposed to an antibody fragment. Specifically, full-length four-chain antibodies include those having heavy and light chains including an Fc region. A full-length heavy chain-only antibody comprises a heavy chain variable domain (for example, VHH) and an Fc region. The constant domain may be a native sequence constant domain (for example, a human native sequence constant domain) or an amino acid sequence variant thereof. In some cases, an intact antibody may have one or more effector functions.

An "antibody fragment" or "antigen-binding fragment" comprises a portion of an intact antibody, preferably the antigen-binding and/or variable region of the intact antibody. Examples of antibody fragments include, but are not limited to, Fab, Fab', F(ab')2 and Fv fragments; diabodies; linear antibodies; single-chain antibody (scFv) molecules; single domain antibodies (such as VHH), and multispecific antibodies formed from antibody fragments. An "Fv" is the smallest antibody fragment containing a complete antigen-recognition and -binding site. This fragment consists of a dimer of one heavy-chain and one light-chain variable region domain in tight, non-covalent association. A "single-chain Fv" also abbreviated "sFv" or "scFv" is an antibody fragment comprising VH and VL antibody domains linked to a single polypeptide chain. Preferably, the scFv polypeptide further comprises a polypeptide linker between the VH and VL domains that allows the scFv to form the desired structure for antigen binding. A "diabody" refers to a small antibody fragment prepared by constructing an sFv fragment with a short linker (about 5 to 10 residues) between the VH and VL domains such that interchain, but not intrachain, pairing of the V domains is achieved, thereby obtaining a bivalent fragment, that is, a fragment with two antigen-binding sites. Bispecific diabodies are heterodimers of two "crossover" sFv fragments in which the VH and VL domains of two antibodies are present on different polypeptide chains.

The term "humanized antibody" is used as a subset of "chimeric antibody".

"Humanized" forms of non-human (for example, llama or Camelidae) antibodies are chimeric antibodies that contain minimal sequence derived from non-human immunoglobulin. In some embodiments, a humanized antibody is a human immunoglobulin (recipient antibody) in which residues from the CDRs of the recipient (as defined below) are replaced with residues from the CDRs of a non-human species (donor antibody), for example, mouse, rat, rabbit, camel, llama, alpaca, or non-human primate having the desired specificity, affinity, and/or capacity.

In some instances, framework ("FR") residues of the human immunoglobulin are replaced with the corresponding non-human residues. In addition, a humanized antibody may comprise residues that are found neither in the recipient antibody nor in the donor antibody. These modifications may be made to further improve antibody performance, for example, binding affinity.

The term "hypervariable region", "HVR", or "HV" when used herein refers to a region of an antibody variable domain that is hypervariable in sequence and/or forms structurally defined loops. Generally, single domain antibodies comprise three HVRs (or CDRs): HVR1 (or CDR1), HVR2 (or CDR2), and HVR3 (or CDR3). HVR3 (or CDR3) is known to display the highest diversity of the three HVRs and to play a unique role in conferring microspecificity to the antibodies. See, for example, Hamers-Casterman et al., Nature 363:446-448 (1993); Sheriff et al., Nature Struct. Biol. 3:733-736 (1996).

The term "complementarity determining region" or "CDR" is used to refer to a hypervariable region as defined by the Kabat system. See Kabat, Elvin A., Sequences of Proteins of Immunological Interest, 5th Ed. Public Health Service, National Institutes of Health, Bethesda, Md. (1991). Kabat complementarity determining regions (CDRs) are based on sequence variability and are most commonly used.

The term "framework" or "FR" residues, as defined herein, are variable-domain residues other than HVR residues.

The term "specific" refers to the selective recognition of an antigen-binding protein (for example, sdAb) for a particular epitope of an antigen.

Natural antibodies are, for example, monospecific. As used herein, the term "multispecific" indicates that an antigen-binding protein has polyepitope specificity (that is, is capable of specifically binding to two, three, or more different epitopes on one biological molecule or specifically binding to epitopes on two, three, or more different biological molecules). As used herein, the term "bispecific" indicates that an antigen-binding protein has two different antigen-binding specificities.

As used herein, the term "monospecific" indicates that an antigen-binding protein has one or more binding sites each binding to the same epitope of the same antigen.

The term "valence" indicates the presence of a specified number of binding sites on an antigen-binding protein. For example, the terms "bivalent", "trivalent", "tetravalent", "pentavalent", and "hexavalent" indicates the presence of two binding sites, three binding sites, four binding sites, five binding sites, and six binding sites on an antigen-binding protein.

The "antibody effector function" refers to those biological activities that are attributable to the Fc region (native sequence Fc region or amino acid sequence variant Fc region) of the antibody, and varies across antibody isotypes. Examples of antibody effector functions include: C1q binding and complement dependent cytotoxicity; Fc receptor binding; antibody-dependent cell-mediated cytotoxicity (ADCC); phagocytosis; down regulation of cell surface receptors (for example, B cell receptor); and B cell activation. The "complement dependent cytotoxicity" or "CDC" refers to the lysis of a target cell in the presence of complement. Activation of the classical complement pathway is initiated by the binding of the first component (C1q) of the complement system to antibodies (of the appropriate subclass) that bind to their cognate antigens. The "antibody-dependent cell-mediated cytotoxicity" or ADCC refers to a form of cytotoxicity in which secreted Ig that binds to Fc receptors (FcR) present on certain cytotoxic cells (for example, natural killer (NK) cells, neutrophils and macrophages) specifically bind these cytotoxic effector cells to antigen-bearing target cells and subsequently kills the target cells with cytotoxins.

The term "Fc region" or "fragment crystallizable region" is used herein to define the C-terminal region of an immunoglobulin heavy chain, including native-sequence Fc regions and variant Fc regions. Suitable native-sequence Fc regions for use in the antibodies described herein include human IgG1, IgG2 (IgG2A, IgG2B), IgG3 and IgG4.

The "binding affinity" generally refers to the strength of the sum of non-covalent interactions between a single binding site of a molecule (for example, antibody) and its binding partner (for example, antigen). As used herein, unless otherwise specified, "binding affinity" refers to intrinsic binding affinity that reflects a 1:1 interaction between members of a binding pair. Binding affinity can be expressed as K_{d}, K_{off}, Kₒₙ, or Kₐ. As used herein, the term equilibrium dissociation constant "K_{D}" or "K_{d}" refers to the dissociation constant of a particular antibody-antigen interaction, describes the concentration of antigen required to occupy one half of all antibody-binding domains present in a solution of antibody molecule at equilibrium, and is expressed in the unit of M. The measurement of K_{D} is performed on the premise that all binding agents are in solution. The dissociation constant (K_{D} or K_{d}) is used as an indicator of the affinity of an antibody for an antigen. For example, easy analysis is possible by the Scatchard method using antibodies marked with various marker agents as well as by using over-the-counter drugs and measurement kits according to the user's manual attached to the kit and the experimental operation method. K_{D} values that can be derived using these methods are expressed in the unit of M (mols).

The "percent (%) amino acid sequence identity" and "homology" for a peptide, polypeptide or antibody sequence is defined as the percentage of amino acid residues in a candidate sequence that are identical to amino acid residues in a specific peptide or polypeptide sequence after sequence alignment and introduction of gaps, and without considering any conservative substitutions as part of sequence identity, if necessary, to achieve maximum percent sequence identity. Alignment for purposes of determining percent amino acid sequence identity may be achieved in a variety of ways that are within the skill of the art, for example, using publicly available computer software such as BLAST, BLAST-2, ALIGN or MEGALIGN^{™} (DNATAR) software. Those skilled in the art can determine suitable parameters for measuring alignment, including any algorithms needed to achieve maximal alignment over the full length of the sequences being compared.

The present invention relates to an antibody comprising a single domain antibody (sdAb) that specifically binds to CD47 (hereinafter referred to as "anti-CD47 sdAb") or antigen-binding fragment thereof, for example, anti-CD47 sdAb, anti-CD47 heavy chain-only antibody (HCAb) (for example, an anti-CD47 sdAb-Fc fusion protein in which an anti-CD47 sdAb is fused to a crystalline fragment (Fc fragment) of human immunoglobulin G (IgG)), or a multispecific antigen-binding protein in which an anti-CD47 sdAb is fused to another sdAb, a full-length four-chain antibody or an antigen binding fragment thereof (for example, Fab or scFv); and preparation and uses of the same.

Accordingly, the present invention provides an antibody comprising an anti-CD47 sdAb or an antigen-binding fragment thereof.

In the present invention, the antibody comprising an anti-CD47 sdAb or an antigen-binding fragment thereof may be an anti-CD47 sdAb or an antigen-binding fragment thereof.

In the present invention, the anti-CD47 sdAb includes CDR1 consisting of an amino acid sequence represented by any one of SEQ ID NOs: 3 and 6; CDR2 consisting of an amino acid sequence represented by any one of SEQ ID NOs: 4 and 7; and CDR3 consisting of an amino acid sequence represented by any one of SEQ ID NOs: 5 and 8.

The CDR sequences are provided in Table 6. In addition, the CDRs may be combined in any combination to produce a number of anti-CD47 sdAbs.

Specifically, the anti-CD47 sdAb may include any CDR among the following:
(1) CDR1 consisting of an amino acid sequence represented by SEQ ID NO: 3;
   CDR2 consisting of an amino acid sequence represented by SEQ ID NO: 4; and
   CDR3 consisting of an amino acid sequence represented by SEQ ID NO: 5; or
(2) CDR1 consisting of an amino acid sequence represented by SEQ ID NO: 6;
   CDR2 consisting of an amino acid sequence represented by SEQ ID NO: 7; and
   CDR3 consisting of an amino acid sequence represented by SEQ ID NO: 8.

In the present invention, the anti-CD47 sdAb may include any suitable sequence for the FR region. Specifically, the FR sequence may be an amino acid sequence shown in Tables 1 to 4 below.

**[Table 1]**

| NO | FR1 | SEQ ID NO: |
|---|---|---|
| 1 | QVQLVESGGGLVQPGGSLNLSCAVS | 11 |
| 2 | QVQLVESGGGLVQPGGSLRLSCTAS | 15 |

**[Table 2]**

| NO | FR2 | SEQ ID NO: |
|---|---|---|
| 1 | VGWARQVPGKGLERVSR | 12 |
| 2 | ISWFRQTPGKEREGVSC | 16 |

**[Table 3]**

| NO | FR3 | SEQ ID NO: |
|---|---|---|
| 1 | EYLDAVKGRFTISRDNAKNTVYLQMNSLKTEDTAVYYC | 13 |
| 2 | NYAASVKGRFTISRDDAQNTVYLFMNDLKPEDTAVYYC | 17 |

**[Table 4]**

| NO | FR4 | SEQ ID NO: |
|---|---|---|
| 1 | WGQGTGVTVSS | 14 |
| 2 | WGKGTLVTVSS | 18 |

More specifically, the anti-CD47 sdAb may include the following FR1, FR2, FR3 and FR4: FR1 consisting of an amino acid sequence represented by any one of SEQ ID NOs: 11 and 15;
FR2 consisting of an amino acid sequence represented by any one of SEQ ID NOs: 12 and 16;
FR3 consisting of an amino acid sequence represented by any one of SEQ ID NOs: 13 and 17; and
FR4 consisting of an amino acid sequence represented by any one of SEQ ID NOs: 14 and 18.
Still more specifically, the anti-CD47 sdAb may include the following FR1, FR2, FR3 and FR4:
   (1) FR1 consisting of an amino acid sequence represented by SEQ ID NO: 11;
      FR2 consisting of an amino acid sequence represented by SEQ ID NO: 12;
      FR3 consisting of an amino acid sequence represented by SEQ ID NO: 13; and
      FR4 consisting of an amino acid sequence represented by SEQ ID NO: 14; or
   (2) FR1 consisting of an amino acid sequence represented by SEQ ID NO: 15;
      FR2 consisting of an amino acid sequence represented by SEQ ID NO: 16;
      FR3 consisting of an amino acid sequence represented by SEQ ID NO: 17; and
      FR4 consisting of an amino acid sequence represented by SEQ ID NO: 18.

In the present invention, the anti-CD47 sdAb may include a VHH domain including the FR region.

Specifically, the anti-CD47 sdAb may include an amino acid sequence represented by any one of SEQ ID NOs: 1 and 2, or a variant thereof having at least 80% (for example, at least arbitrary 80%, 88%, 90%, 91%, 92%, 93%, 94%, 95%, 96%, 97%, 98% or 99%) sequence homology to the amino acid sequence.

In the present invention, the anti-CD47 sdAb may be monomeric or multimeric. In a case of being multimeric, the anti-CD47 sdAb may be multispecific and multivalent (for example, bispecific and bivalent), for example, including two or more different anti-CD47 sdAbs described herein; or may be monospecific and multivalent (for example, bivalent), including two or more copies of the same anti-CD47 sdAb. In addition, the sdAbs may be fused to each other via a peptide linker. In some embodiments, the peptide linker includes flexible residues (for example, glycine and serine) so that adjacent domains are free to move relative to each other. For example, a glycine-serine doublet may be a suitable peptide linker. In addition, the peptide linker may be of any suitable length. In some embodiments, the peptide linker is at least about arbitrary 1, 2, 3, 4, 5, 6, 7, 8, 9, 10, 11, 12, 13, 14, 15, 16, 17, 18, 19, 20, 25, 30, 35, 40, 50, 75, 100 or more amino acids in length.

In the present invention, the anti-CD47 sdAb binds to an epitope of CD47.

In addition, the K_{D} of binding between the anti-CD47 sdAb and CD47 may be 10⁻⁶ M to 10⁻¹² M, 10⁻⁶ M to 10⁻¹¹ M, 10⁻⁶ M to 10⁻¹⁰ M, 10⁻⁶ M to 10⁻⁹ M, or 10⁻⁶ M to 10⁻⁸ M.

In addition, the EC₅₀ of the anti-CD47 sdAb may be less than 500 nM in FACS analysis, specifically 0.1 nM to 500 nM, 0.1 nM to 400 nM, 0.1 nM to 300 nM, 0.1 nM to 200 nM, 0.1 nM to 100 nM, 0.1 to 50 nM, 0.1 to 10 nM, 1 nM to 500 nM, 1 nM to 400 nM, 1 nM to 300 nM, 1 nM to 200 nM, 1 nM to 100 nM, 1 to 50 nM, or 1 to 10 nM.

The single domain antibody (sdAb) according to the present invention may include, without limitation, engineered domains and single domain scaffolds in addition to heavy chain variable domains from heavy chain-only antibodies (for example, VHH in Camelidae (variable domain of the heavy chain of a heavy chain antibody)); light chains derived from conventional four-chain antibodies; binding molecules naturally devoid of a single domain (for example, VH or VL); humanized heavy chain only antibodies; human single domain antibodies produced by transgenic mice or rats expressing human heavy chain segments; and those derived from antibodies. The sdAb may be derived from any species, including but not limited to mouse, rat, human, camel, llama, lamprey, fish, shark, goat, rabbit, and bovine. The sdAb may also include naturally occurring sdAb molecules from species other than Camelidae.

In addition, the sdAb is derived from naturally occurring single domain antigen binding molecules known as heavy chain antibodies devoid of light chains. Such single domain molecules are disclosed, for example, in WO 94/04678 and Hamers-Casterman, et al., (1993) Nature 363:446-448. Variable domains derived from heavy chain molecules naturally devoid of light chains are known herein as VHHs to distinguish these from the conventional VHs of four chain immunoglobulins. Such VHH molecules may be derived from antibodies produced in species of Camelidae such as camels, llamas, vicuñas, dromedary camels, alpacas and guanacos. Species other than Camelidae can produce heavy chain molecules naturally devoid of light chains, and such VHHs are within the scope of the present disclosure.

In addition, the sdAb may be recombinant, CDR-grafted, humanized, camelized, de-immunized and/or produced in vitro (for example, selected by phage display). In some embodiments, the amino acid sequence of a framework region may be altered by "camelization" of specific amino acid residues in the framework region. Camelization refers to the replacement or substitution of one or more amino acid residues in the amino acid sequence of the (naturally occurring) VH domain from a conventional four-chain antibody with one or more amino acid residues occurring at the corresponding position(s) in the VHH domain of the heavy chain antibody, and may be performed in a way known in the art.

In addition, the sdAb may be a human sdAb produced by a transgenic mouse or rat expressing a human heavy chain segment. See, for example, patents US 20090307787 A1, US 8,754,287, US 20150289489 A1, US 20100122358 A1, and WO 2004049794.

In addition, naturally occurring VHH domains for a particular antigen or target may be obtained from a (naive or immune) library of Camelid VHH sequences. Such methods may or may not involve screening of such a library using the antigen or target, or at least a portion, fragment, antigenic determinant or epitope thereof using one or more screening techniques known per se. Such libraries and techniques are described in, for example, patents WO 99/37681, WO 01/90190, WO 03/025020 and WO 03/035694. Alternatively, improved synthetic or semi-synthetic libraries derived from (naive or immune) VHH libraries, for example, VHH libraries obtained from (naive or immune) VHH libraries by techniques, for example, random mutagenesis and/or CDR shuffling as described in, for example, patent WO 00/43507 may be used.

In addition, the sdAb may be produced from conventional four-chain antibodies. See, for example, Ward et al., Nature 1989 Oct. 12; 341 (6242): 544-6, Holt et al., Trends Biotechnol., 2003, 21(11):484-490; patent WO 06/030220; and patent WO 06/003388.

In addition, the sdAb according to the present invention may be a chimeric antibody. Certain chimeric antibodies are described in, for example, patent US 4,816,567; and Morrison et al., Proc. Natl. Acad. Sci. USA, 81:6851-6855 (1984)). In some embodiments, a chimeric antibody may comprise a non-human variable region (for example, a variable region derived from a species of Camelidae, such as a llama) and a human constant region. In addition, a chimeric antibody may be humanized. Typically, a non-human antibody is humanized to reduce immunogenicity to humans but maintain the specificity and affinity of the parental non-human antibody. Generally, a humanized antibody comprises one or more variable domains in which the HVRs, for example, CDRs (or portions thereof) are derived from non-human antibodies and the FRs (or portions thereof) are derived from human antibody sequences. A humanized antibody optionally also will comprise at least a portion of a human constant region. In some embodiments, some FR residues in a humanized antibody are substituted with the corresponding residues from a non-human antibody (for example, an antibody from which the HVR residues are derived), for example, in order to restore or improve antibody specificity or affinity.

In the present invention, the antibody comprising an anti-CD47 sdAb or an antigen-binding fragment thereof may be an anti-CD47 HCAb or an antigen-binding fragment thereof.

Specifically, the anti-CD47 HCAb is one in which the anti-CD47 sdAb described herein is fused to one or more CH2 and/or CH3 domains, for example, Fc fragments.

The CH2 and/or CH3 domains are derived from immunoglobulins. The immunoglobulin may be IgA, IgD, IgE, IgG or IgM, specifically IgG. In some embodiments, the anti-CD47 HCAb may comprise an Fc fragment of IgG, such as IgG1, IgG2, IgG3 or IgG4, and the Fc fragment may be human Fc, for example, human IgG1 (hIgG1) Fc, hIgG2 Fc, hIgG3 Fc or hIgG4 Fc.

The anti-CD47 HCAb may be monomeric or multimeric. In a case of being multimeric, the anti-CD47 HCAb may be multispecific and multivalent (for example, bispecific and bivalent), for example, including two or more different anti-CD47 sdAbs described herein; or may be monospecific and multivalent (for example, bivalent), including two or more copies of the same anti-CD47 sdAb.

In the present invention, the anti-CD47 sdAb and the CH2 and/or CH3 domains, specifically the Fc fragments, may be fused via a peptide linker. The length, degree of flexibility and/or other properties of the peptide linker may have some effects on properties, including but not limited to affinity, specificity or binding capacity for one or more particular antigens or epitopes. For example, a longer peptide linker may be chosen to ensure that two adjacent domains do not sterically interfere with each other. In some embodiments, the peptide linker includes flexible residues (for example, glycine and serine) so that adjacent domains are free to move relative to each other. For example, a glycine-serine doublet may be a suitable peptide linker. In addition, the peptide linker may be of any suitable length. In some embodiments, the peptide linker is at least about arbitrary 1, 2, 3, 4, 5, 6, 7, 8, 9, 10, 11, 12, 13, 14, 15, 16, 17, 18, 19, 20, 25, 30, 35, 40, 50, 75, 100 or more amino acids in length.

In addition, the peptide linker may have a naturally occurring sequence or a non-naturally occurring sequence. For example, a sequence derived from the hinge region of a heavy chain-only antibody may be used as a linker. See, for example, patent WO 1996/34103. In some embodiments, the peptide linker may be hIgG1 hinge, hIgG2 hinge, hIgG3 hinge, hIgG4 hinge or variants thereof.

In the present invention, the anti-CD47 HCAb may comprise an amino acid sequence represented by any one of SEQ ID NOs: 9, 10 and 19, or a variant thereof having at least 80% (for example, at least arbitrary 80%, 88%, 90%, 91%, 92%, 93%, 94%, 95%, 96%, 97%, 98% or 99%) sequence homology to the amino acid sequence.

In the present invention, the antibody comprising an anti-CD47 sdAb or an antigen-binding fragment thereof may be a multispecific antigen-binding protein (MABP) in which an anti-CD47 sdAb is fused to another sdAb, a full-length four-chain antibody or an antigen binding fragment thereof (for example, a bispecific antigen-binding protein (BABP) having an anti-CD47 sdAb fused thereto (hereinafter referred to as anti-CD47 BABP)) or an antigen-binding fragment thereof.

The anti-CD47 BABP comprises (a) a first antigen-binding moiety including the anti-CD47 sdAb described herein; and (b) a second antigen-binding moiety that specifically binds to a second epitope.

The second epitope may be an antigen other than CD47, or a second epitope on CD47.

The second antigen-binding moiety may be a full-length antibody, Fab, Fab', (Fab')2, Fv, single chain Fv (scFv), scFv-scFv, minibody, diabody or second sdAb. In addition, the second antigen-binding moiety may include a heavy chain including VH and a light chain including VL. In some embodiments, the first antigen-binding moiety may be fused to the second antigen-binding moiety at the N-terminus of the heavy chain, the N-terminus of the light chain, the N-terminus of the Fc region, the C-terminus of the heavy chain, or the C-terminus of the light chain. In some embodiments, the second antigen-binding moiety may include Fab or scFv. In some embodiments, the first antigen-binding moiety may be fused to the second antigen-binding moiety at the C-terminus of Fab or scFv. In some embodiments, the second antigen-binding moiety may include a full-length four-chain antibody. In some embodiments, the first antigen-binding moiety may be fused to the second antigen-binding moiety via a peptide linker. In some embodiments, the second antigen-binding moiety may include an Fc region, for example, IgG1 Fc, IgG2 Fc, IgG3 Fc or IgG4 Fc.

The anti-CD47 MABP comprises at least two antigen-binding moieties that specifically bind to at least two different epitopes. Some of the at least two antigen-binding moieties may be identical as long as the MABP has binding sites for two different epitopes. In addition, the anti-CD47 MABP may comprise any one of 1, 2, 3, 4, 5, 6, 7, 8, or more different antigen-binding moieties, each including the anti-CD47 sdAb described herein.

In addition, the anti-CD47 MABP may have any suitable number of valences with respect to the first epitope and/or the second epitope of CD47, and any suitable number of specificities. In some embodiments, the anti-CD47 MABP may be bivalent, trivalent, tetravalent, pentavalent, hexavalent, or higher valent with respect to CD47. In some embodiments, MABP may be trispecific, and may be quadrispecific.

Techniques for preparing multispecific antibodies according to the present invention include, but are not limited to, recombinant co-expression of two immunoglobulin heavy-light chain pairs with different specificities (for example, Milstein and Cuello, Nature 305: 537 (1983)); WO 93/08829; Traunecker et al., EMBO J. 10: 3655 (1991)), and "knob-in-hole" engineering (for example, patent US 5,731,168). Multispecific antibodies may also be prepared by engineering electrostatic steering effects for preparing antibody Fc-heterodimeric molecules (WO 2009/089004A1); cross-linking of two or more antibodies or fragments (for example, patent US 4676980, and Brennan et al., Science, 229: 81 (1985)); use of leucine zippers to produce bi-specific antibodies (for example, Kostelny et al., J. Immunol., 148(5):1547-1553 (1992)); use of "diabody" technique for preparing bispecific antibody fragments (for example, Hollinger et al., Proc. Natl. Acad. Sci. USA, 90:6444-6448 (1993)); and use of single-chain Fv (sFv) dimers (for example, Gruber et al., J. Immunol., 152:5368 (1994)); and, for example, preparation of trispecific antibodies as described in Tutt et al., J. Immunol. 147: 60 (1991); and creation of polypeptides including tandem single-domain antibodies (for example, patent US 20110028695; and Conrath et al., J. Biol. Chem., 2001; 276(10):7346-50).

In the present invention, the antibody comprising an anti-CD47 sdAb or an antigen-binding fragment thereof includes an amino acid sequence variant. Amino acid sequence variants of an antibody may be prepared by introducing suitable modifications to the nucleic acid sequence encoding the antibody or by peptide synthesis. Such modifications include, for example, deletions, and/or insertions and/or substitutions of residues within the amino acid sequence of the antibody. Any combination of deletions, insertions, and substitutions may be made to result in a final construct, provided that the final construct retains the desired characteristics, for example, antigen-binding. In some embodiments, substitutions, insertions, or deletions may occur within one or more hypervariable regions (HVRs) as long as such alterations do not substantially reduce the ability of the antibody to bind to an antigen. For example, conservative alterations that do not substantially reduce binding affinity may be made in the HVRs. Such changes may be outside of HVR "hotspots" or CDRs.

In addition, the amino acid substitution may be at least one (for example, arbitrary 1, 2, 3, 4, 5, 6, 7, 8, 9, or 10) amino acid substitution. In addition, the at least one amino acid substitution may be a conservative substitution, and may be a substitution with a non-genetically encoded amino acid or a synthetic amino acid. In some embodiments, the amino acid substitution may be in a CDR region, and may include at least one (for example, arbitrary 1, 2, 3, or 4) amino acid substitution in CDR1, CDR2 and/or CDR3. In some embodiments, the amino acid substitution may be in an FR region, and may include at least one (for example, arbitrary 1, 2, 3, 4, 5 or 6) amino acid substitution in FR1, FR2, FR3 and/or FR4.

In addition, the amino acid sequence insertion includes amino- and/or carboxyl-terminal fusions ranging in length from one residue to a polypeptide containing 100 or more residues, as well as insertions of single or multiple amino acid residues into the sequence. Examples of terminal insertions include antibodies having an N-terminal methionyl residue. Other insertional variants of antibody molecule may include the fusion to the N- or C-terminus of the antibody to an enzyme (for example, for ADEPT) or a polypeptide that increases the serum half-life of the antibody.

In addition, one or more amino acid modifications may be introduced into the Fc region of the antibody comprising an anti-CD47 sdAb or an antigen-binding fragment thereof (for example, anti-CD47 HCAb, or anti-CD47 MABP), which is provided herein, thereby producing an Fc region variant. The Fc region variant may comprise a human Fc region sequence (for example, human IgG1, IgG2, IgG3 or IgG4 Fc) including an amino acid modification (for example, substitution) at one or more amino acid positions.

In the present invention, the antibody comprising an anti-CD47 sdAb or an antigen-binding fragment thereof may be linked to, fused to, conjugated to (for example, covalently or non-covalently), or otherwise associated with a diagnostic moiety or biocompatibility modifier. For example, peptides or polypeptides (for example, biotoxins, biomarkers, and purification tags), proteins, polymers, nucleic acid molecules, small molecules, mimics, synthetic drugs, inorganic molecules, organic molecules, or radioisotopes may be conjugated or associated.

In addition, the antibody comprising an anti-CD47 sdAb or an antigen-binding fragment thereof may be conjugated to or associated with a diagnostic or detectable agent, marker or reporter, which may be a biological molecule (for example, peptide or nucleotide), a small molecule, a fluorophore, or a radioisotope. The labeled modulator may be useful for monitoring the development or progression of a disease associated with CD47, such as cancer, or as part of a clinical trial procedure to determine the efficacy (namely, theragnosis) of a particular therapy comprising the antibody disclosed herein or to determine a future course of treatment. Such a marker or reporter may also be useful for purifying the antibody disclosed herein.

In addition, the antibody comprising an anti-CD47 sdAb or an antigen-binding fragment thereof may be conjugated to an immunomodulator, cytokine, cytotoxic agent, chemotherapeutic agent, diagnostic agent, antiviral agent, antimicrobial agent or drug. Accordingly, the present invention provides an antibody conjugate comprising the antibody comprising an anti-CD47 sdAb or an antigen-binding fragment thereof according to the present invention, which is conjugated to an immunomodulator, cytokine, cytotoxic agent, chemotherapeutic agent, diagnostic agent, antiviral agent, antimicrobial agent or drug.

The present invention also provides a nucleic acid molecule encoding the antibody comprising an anti-CD47 sdAb or an antigen-binding fragment thereof disclosed herein; an expression vector comprising the nucleic acid molecule, and a host cell transformed with the expression vector.

The present invention also provides a method for producing an antibody or an antigen-binding fragment thereof, which comprises (a) culturing the host cell under conditions that allow expression of the antibody; and (b) recovering the expressed antibody or an antigen-binding fragment thereof.

In the present invention, DNA encoding the antibody comprising an anti-CD47 sdAb or an antigen-binding fragment thereof disclosed herein can be readily isolated and sequenced using conventional procedures (for example, by using oligonucleotide probes capable of binding specifically to genes encoding antibody heavy and light chains). Isolated and subcloned hybridoma cells (or phage or yeast-derived colonies) may serve as a preferred source of such DNA. More particularly, isolated DNA (which may be modified) can be used to clone constant and variable region sequences for the preparation of antibodies.

An exemplary method involves extraction of RNA from selected cells, conversion to cDNA, and amplification by PCR using antibody specific primers. Suitable primers are well known in the art and are readily available from a large number of commercial sources, as exemplified herein. In order to express a recombinant human or non-human antibody isolated by screening of a combinatorial library, DNA encoding the antibody is cloned into a recombinant expression vector and introduced into a host cell, including mammalian cells, insect cells, plant cells, yeast, and bacteria. In some embodiments, a modulator is introduced into monkey COS cells, NS0 cells, Chinese Hamster Ovary (CHO) cells, or myeloma cells that do not otherwise produce the desired construct, and the antibody is expressed by the modulator.

In the present invention, the nucleic acid molecule is present in a vector, where appropriate, together with a promoter controlling the expression of the nucleic acid. The vector is used in its most general sense, and includes any intermediate vehicle for the nucleic acid which allows the nucleic acid to be introduced into, for example, prokaryotic and/or eukaryotic cells and, where appropriate, integrated into the genome. Vectors of this kind are preferably replicated and/or expressed intracellularly. The vector may include a plasmid, phagemid, bacteriophage or viral genome. The plasmid generally refers to a construct of extrachromosomal genetic material capable of replicating independently of chromosomal DNA, typically a circular DNA duplex.

Expression vectors containing antibody coding sequences and appropriate transcriptional and translational control signals may be constructed using methods well known to those skilled in the art. These methods include, for example, in vitro recombinant DNA techniques, synthetic techniques, and in vivo genetic recombination.

In the present invention, the host cell or recombinant host cell refers to a cell into which an expression vector is introduced. The recombinant host cell and host cell refer to a particular subject cell as well as the progeny of such a cell. Such progeny may not in fact be identical to the parent cell as certain modifications may occur in subsequent generations due to mutations or environmental influences, but are still included within the scope of the term host cell as used herein. Such cells may include vectors as described above.

In addition, using molecular biology techniques and current protein expression methodologies recognized in the art, substantial amounts of the antibody disclosed herein may be produced. More specifically, the nucleic acid molecule encoding such an antibody may be incorporated into well-known and commercially available protein production systems, including various types of host cells to provide preclinical, clinical, or commercial amounts of the desired pharmaceutical product. In some embodiments, the nucleic acid molecule encoding the antibody is engineered into a vector or expression vector that affords efficient integration into a selected host cell and subsequent high expression levels of the antibody.

Preferably, the nucleic acid molecule encoding the antibody disclosed herein and the vector comprising the nucleic acid molecule may be used for transfection of suitable mammalian, plant, bacterial or yeast host cells, but prokaryotic systems may also be used. Transfection may be accomplished by any known method for introducing a polynucleotide into a host cell. Methods for introducing heterologous polynucleotides into mammalian cells are well known in the art, and include dextran-mediated transfection, calcium phosphate precipitation, polybrenemediated transfection, protoplast fusion, electroporation, encapsulation of polynucleotide(s) in liposomes, and direct microinjection of DNA into nuclei. In addition, the nucleic acid molecule may be introduced into mammalian cells by means of viral vectors. Methods of transforming mammalian cells are well known in the art. Methods of transforming plant cells are also well known in the art, and examples thereof include Agrobacteriummediated transformation, biolistic transformation, direct injection, electroporation, and viral transformation. Methods of transforming bacterial and yeast cells are also well known in the art.

A variety of commercially available host-expression vector systems may be used to express the antibody disclosed herein. Such host-expression systems not only represent a vehicle in which a coding sequence of interest can be expressed and subsequently purified, but also a cell capable of expressing in situ a molecule of the present invention when transformed or transfected with an appropriate nucleotide coding sequence. Such systems include microorganisms, such as bacteria (for example, E. coli, B. subtilis, and streptomyces), transformed with recombinant bacteriophage DNA, plasmid DNA or cosmid DNA expression vectors containing modulator coding sequences; yeast (for example, Saccharomyces, Pichia) transfected with recombinant yeast expression vectors containing modulator coding sequences; insect cell systems infected with recombinant virus expression vectors (for example, baculovirus) containing modulator coding sequences; plant cell systems (for example, Nicotiana, Arabidopsis, duckweed, corn, wheat, and potato) infected with recombinant viral expression vectors (for example, cauliflower mosaic virus, CaMV; tobacco mosaic virus, and TMV) or transfected with recombinant plasmid expression vectors (for example, Ti plasmid) containing modulator coding sequences; or mammalian cell systems (for example, COS, CHO, BHK, 293, 3T3 cells) retaining recombinant expression constructs containing promoters derived from the genome of mammalian cells (for example, metallothionein promoter) or derived from mammalian viruses (for example, adenovirus late promoter; vaccinia virus 7.5K promoter), but are not limited thereto.

When the antibody disclosed herein is produced by recombinant expression or any one of the other techniques disclosed herein, the antibody may be purified by any method known in the art for the purification of immunoglobulins, or may more generally be purified by any other standard technique for the purification of proteins.

The present invention also provides a pharmaceutical composition for prevention or treatment of cancer, comprising the antibody comprising an anti-CD47 sdAb or an antigen-binding fragment thereof disclosed herein or an antibody conjugate comprising the antibody or an antigen-binding fragment thereof as an active ingredient.

The present invention also provides a method for preventing or treating cancer, which comprises administering a pharmaceutical composition comprising the antibody comprising an anti-CD47 sdAb or an antigen-binding fragment thereof disclosed herein or an antibody conjugate comprising the antibody or an antigen-binding fragment thereof to an individual in a pharmaceutically effective amount.

The present invention also provides the use of the antibody comprising an anti-CD47 sdAb or an antigen-binding fragment thereof disclosed herein or an antibody conjugate comprising the antibody or an antigen-binding fragment thereof for the prevention or treatment of cancer.

In the present invention, the cancer is a cancer that requires activation of T cells by blocking the activity of immune checkpoint proteins, and may be selected, for example, from the group consisting of melanoma, lung cancer, liver cancer, glioblastoma, ovarian cancer, colorectal cancer, head and neck cancer, bladder cancer, renal cell cancer, stomach cancer, breast cancer, metastatic cancer, prostate cancer, pancreatic cancer, non-Hodgkin's lymphoma, Hodgkin's lymphoma, multiple myeloma, leukemia, lymphoma, myelodysplastic syndrome, acute lymphoblastic leukemia, acute myelogenous leukemia, chronic lymphocytic leukemia, chronic myelogenous leukemia, solitary myeloma and aplastic anemia, but is not limited thereto.

In the present invention, since the contents of the antibody comprising an anti-CD47 sdAb or an antigen-binding fragment thereof or the antibody conjugate comprising the antibody or an antigen-binding fragment thereof are the same as described above, specific descriptions thereof use the above contents, and only the unique configuration of the pharmaceutical composition and use will be described below.

The pharmaceutical composition according to the present invention may comprise one or more (for example, two or three) antibodies comprising an anti-CD47 sdAb or antigen-binding fragments thereof described herein or drug conjugates comprising the antibodies or antigen-binding fragments thereof.

By administering the pharmaceutical composition according to the present invention to an individual, specifically a cancer patient, cancer may be prevented or treated.

In addition, the pharmaceutical composition according to the present invention may be formulated as desired using techniques recognized in the art depending on the form of the antibody described herein, the intended way of delivery, and numerous other variables. In addition, the pharmaceutical composition may be formulated to contain suitable pharmaceutically acceptable carriers, including excipients and auxiliaries, which are well known in the art and are relatively inert substances that facilitate administration or assist in the processing of the active compounds into formulations that are pharmaceutically optimized for delivery. A variety of pharmaceutically acceptable carriers, including, for example, vehicles, adjuvants, and diluents, are readily available from numerous commercial sources. In addition, classes of pharmaceutically acceptable auxiliary substances, such as pH adjusting and buffering agents, tonicity adjusting agents, stabilizers, and wetting agents, are also available. Certain non-limiting exemplary carriers include saline, buffered saline, dextrose, water, glycerol, ethanol, and combinations thereof.

In addition, the pharmaceutical composition according to the present invention may be formulated for enteral, parenteral or topical administration. In fact, all three types of formulation may be used simultaneously to achieve systemic administration of the active ingredient. Excipients as well as formulations for parenteral and non-parenteral drug delivery are known in the art. Formulations suitable for parenteral administration include aqueous solutions of the active compounds in water-soluble form, for example, water-soluble salts. In addition, suspensions of the active compounds appropriate for oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspensions, and examples thereof include sodium carboxymethyl cellulose, sorbitol, and/or dextran. Optionally, suspensions may also contain stabilizers. Liposomes may also be used to encapsulate agents for delivery to cells.

Formulations suitable for enteral administration include hard or soft gelatin capsules, pills, tablets including coated tablets, elixirs, suspensions, syrups or inhalants and controlled release forms thereof.

In general, the antibody disclosed herein may be administered in vivo to a subject in need thereof by a variety of routes including, but not limited to, oral, intravenous, intraarterial, subcutaneous, parenteral, intranasal, intramuscular, intracardiac, intraventricular, intratracheal, buccal, rectal, intraperitoneal, intradermal, topical, transdermal, and intrathecal, or otherwise by implantation or inhalation. Appropriate formulations and routes of administration may be selected depending on the intended use and treatment regimen.

The pharmaceutical composition according to the present invention is administered in a pharmaceutically effective amount for the treatment or prevention of cancer. The pharmaceutically effective amount refers to an amount of an antibody or pharmaceutical composition comprising the same that will elicit a biological or medical response in a subject, as sought by a physician or other clinician. In addition, multiple doses of the antibody or pharmaceutical composition comprising the same may be administered at a certain frequency to achieve an amount of therapy that has a prophylactic and/or therapeutic effect.

The pharmaceutically effective amount typically depends on the weight and physical condition of the subject to be treated, the breadth of the condition to be treated, and the age of the subject to be treated. In general, the antibody disclosed herein may be administered in an amount ranging from about 10 ng/kg body weight to about 100 mg/kg body weight, from about 50 µg/kg body weight to about 5 mg/kg body weight, from about 100 µg/kg body weight to about 10 mg/kg body weight, from about 100 µg/kg body weight to about 20 mg/kg body weight, or from 0.5 mg/kg body weight to about 20 mg/kg body weight per dose, but is not limited thereto. In addition, the antibody may be administered at a dose of at least about 100 µg/kg body weight, at least about 250 µg/kg body weight, at least about 750 µg/kg body weight, at least about 3 mg/kg body weight, at least about 5 mg/kg body weight, or at least about 10 mg/kg body weight, but is not limited thereto.

In addition, the pharmaceutical composition according to the present invention may be administered in a dose of about 100 mg to about 10,000 mg, about 200 mg to about 9,000 mg, about 300 mg to about 8,000 mg, about 400 mg to about 7,000 mg, or 500 mg to 5,000 mg, but is not limited thereto.

The pharmaceutical composition according to the present invention is usually administered to a patient multiple times. Exemplary treatment regimens entail administration once every two weeks, once a month, or once every 3 to 6 months. For example, the patient may receive the antibody (for example, as an intravenous formulation) once every 4 weeks, for example, every 28 days as a cycle. The frequency of administration may be adjusted depending on the pharmacokinetic profile of the antibody in the patient. For example, the half-life of the antibody may require a two week frequency of administration. In some methods, two or more antibodies with different binding specificities may be administered simultaneously, in which case the dosage of each antibody administered is within the ranges given.

The dosage and frequency depend on the half-life of the antibody in the patient. In general, human antibodies have the longest half-life, followed by humanized antibodies, chimeric antibodies, and non-human antibodies. The dosage and frequency of administration may vary depending on whether the treatment is prophylactic or therapeutic.

The duration of treatment regimen depends on the disease to be treated, the age and condition of the patient, the stage and type of the patient's disease, how the patient responds to treatment, and the like. The clinician may closely monitor the effectiveness of the therapy and make any adjustment as needed. When agents are used in combination, the two or more therapeutic agents are administered simultaneously or sequentially in any order, that is, the antibody disclosed herein may be administered prior to administration of the second therapeutic agent, concurrently with the second therapeutic agent, or subsequent to administration of the second therapeutic agent.

The present invention also provides a method for detecting CD47 or determining an amount of CD47 in a sample, which comprises:
(i) bringing the sample into contact with the antibody comprising an anti-CD47 sdAb or an antigen-binding fragment thereof disclosed herein; and
(ii) detecting formation of a complex between the antibody or an antigen-binding fragment thereof and CD47 or determining an amount of the complex.

In the present invention, since the contents of the antibody comprising an anti-CD47 sdAb or an antigen-binding fragment thereof or the antibody conjugate comprising the antibody or an antigen-binding fragment thereof are the same as described above, specific descriptions thereof use the above contents.

In the present invention, the sample may be a cell sample, that is, a sample containing cells such as cancer cells.

The antibody or antigen-binding fragment of the present invention may be used to detect CD47 or CD47 expressing cells or to determine the amount of CD47 or CD47 expressing cells. By detecting or determining the amount of complex between CD47 and the antibody or antigen-binding fragment of the present invention, CD47 or CD47 expressing cells are detected or the amount of CD47 or CD47 expressing cells is determined. Formation of the complex indicates the presence of CD47 or CD47 expressing cells. Such detection or determination of the amount may be performed by a large number of methods, including but not limited to immunodetection using the antibody or antigen-binding fragment of the present invention. Methods in which antibodies are used to detect peptides or proteins are well known and include ELISA, competitive binding assays, and similar methods. In general, such assays utilize a label provided for detection, for example, indicator enzymes, radiolabels, fluorophores, or a target peptide bound directly or indirectly to the paramagnetic particles, or an antibody or antibody fragment that specifically binds to a protein. The methods of the present invention allow quantitative and/or qualitative evaluations, for example, absolute and/or relative evaluations of the CD47 level or the level of CD47 expressing cells.

### [Examples]

Hereinafter, the present invention will be described in detail by way of Examples and Experimental Examples.

However, the following Examples and Experimental Examples are merely illustrative of the present invention, and the contents of the present invention are not limited to the following Examples.

### <Example 1> Immunization and blood collection

Human CD47 protein as an immune antigen was mixed with an immune adjuvant (GERBU), and one alpaca was inoculated with the mixture three times by intramuscular injection for immunization. The immunization was performed three times, and on day 14 after the last immunization, 10 ml of blood was collected from the alpaca and the immune response was analyzed by ELISA. To determine antibody production, the immune antigen was dispensed into a 96-well microplate at a concentration of 1 ug/ml using a coating buffer and coating was performed overnight at 4°C. After the 96-well microplate was washed with PBST three times, blocking was performed through treatment with 5% skim milk at room temperature for 2 hours to inhibit non-specific binding. Thereafter, after washing with PBST three times, serum samples obtained before immunization (day 0) and 14 days (day 14), 28 days (day 28), and 42 days (day 42) after immunization were treated at each dilution concentration. Thereafter, the 96-well microplate was washed with PBST five times, then the reaction with goat anti-Llama IgG HRP antibody was conducted at room temperature for 1 hour, and whether the antibody was bound to the immune antigen was examined by TMB reaction.

### <Example 2> Library construction and evaluation

An immune library was constructed by amplifying a gene encoding a single domain antibody that bound to the immune antigen identified in <Example 1>. For library construction, peripheral blood mononuclear cells (PBMC) were isolated from blood using Ficoll. A gene fragment encoding the single domain antibody was amplified from total RNA extracted from the isolated peripheral blood mononuclear cells (PBMC) using specific primers, and cloned into a pComb3x vector. The size of the constructed immune library was 5.4 × 10⁸.

### <Example 3> Library amplification

The immune library constructed in <Example 2> was transformed into the XL1-blue strain. The transformed XL1-blue strain was added to 10 ml of 2x YT medium containing 2% glucose and 100 µg/ml ampicillin and cultured in a shaker at 37°C. Culture was performed until the absorbance reached 0.5 at OD₆₀₀, and M13K07 phage (Invitrogen) was added to be 1 × 10¹¹ pfu/ml. Thereafter, static culture was performed at 37°C for 30 minutes, and then additional culture was performed for 30 minutes at 200 rpm in a shaker at 37°C. The culture solution was centrifuged at room temperature and 4,000 rpm for 15 minutes to remove the supernatant. Thereafter, 10 ml of 2x YT medium containing 100 µg/ml ampicillin and 50 µg/ml kanamycin was added to resuspend the pellet in the culture solution, and culture was performed overnight at 250 rpm in a shaking incubator at 30°C. Thereafter, centrifugation was performed at 4°C and 4,000 rpm for 30 minutes. The supernatant was precipitated using the PEG precipitation method, and centrifugation was performed at 4°C and 12,000 rpm for 30 minutes. The pellet was resuspended in PBS, centrifugation was performed at 4°C and 13,000 rpm for 5 minutes, and the supernatant was transferred to a new tube and stored at 4°C until use.

### <Example 4> Bio-panning

To select a single domain antibody specific to an immune antigen, the immune antigen was dispensed into a 96-well microplate at a concentration of 5 ug/ml using a coating buffer and coating was performed overnight at 4°C. The library to be used in the experiment for selecting a single domain antibody (the library of <Example 3>) was dispensed into a 96-well microplate and reacted at room temperature for 30 minutes. Thereafter, the operation of transferring the library to a new well and conducting the reaction at room temperature for 30 minutes was repeated four times. This operation was performed to decrease the amount of the library that non-specifically bound to the microplate well. The library was transferred to a 1.7 ml tube and stored at 4°C until use. After the microplate coated with the immune antigen was washed five times with PBST, 5% skim milk was added, and blocking was performed at room temperature for 2 hours. Thereafter, washing with PBST was performed five times, then the library with a decreased non-specific binding rate was dispensed together with binding solution (2.5% skim milk, 0.5% tween 20) at 5 × 10¹² virions/well, and the reaction was conducted at room temperature for 30 minutes. Thereafter, washing with a washing solution (PBS, 0.5% tween 20) was performed ten times, and then washing with PBST was additionally performed three times. The single domain antibody specifically bound to the immune antigen was selectively eluted by adding 5 µg of immune antigen per well and then conducting the reaction at room temperature and 500 rpm for 30 minutes. XL-1 blue cells in logarithmic growth phase were infected with the eluted phage, and then plated on 2x YT agar medium. For panning for the second screening, the procedure was repeated under the same conditions as above. Each single phage clone produced on the agar medium was amplified and screened using FACS.

### <Example 5> Phage screening

To induce transient overexpression of immune antigen in Expi-CHO cells, a gene encoding an immune antigen was inserted into a pCMV6-GFP vector and a pCMV6-immune antigen-GFP plasmid was constructed. Expi-CHO cells were washed with DPBS and then centrifuged at room temperature and 1,200 rpm for 3 minutes. The supernatant was removed, the cells were resuspended in 2% skim milk, and blocking was performed at 4°C for 30 minutes. The cells were centrifuged at room temperature and 1200 rpm for 3 minutes to remove the supernatant, then washed with DPBS two times, and dispensed into a 96-well microplate at 3 × 10⁵ cells/100 µl/well. A monoclonal phage was added to each well, the reaction was conducted at 4°C for 1 hour, and then washing with DPBS was performed two times. An antibody (M13 major coat protein Alexa Flour 647(Santacruz)) that bound specifically to the phage was dispensed into the cells, and the reaction was conducted at 4°C for 30 minutes while light was blocked. The cells were washed with DPBS two times, resuspended in fresh DPBS, and subjected to FACS analysis using an Accuri C6 (BD) instrument. Screened clones were selected using a FACS system, and sequencing was performed.

### <Example 6> Expression and purification of single domain antibody having human IgG Fc domain fused thereto

The clones selected in <Example 5> were cloned into TGEX-Fc (IgG1) or TGEX-Fc (IgG4) expression vectors. For the expression of a single domain antibody having a human IgG Fc domain fused thereto, Expi-CHO cells with a viability of 95% to 99% were counted, and 7 × 10⁶ cells were added to 25 ml of culture medium (Expi-CHO expression medium (Gibco)) and cultured overnight at 125 rpm in a 37°C shaking incubator maintained at 8% CO₂. Thereafter, a mixture of 20 µg of plasmid DNA encoding a single domain antibody having a human IgG Fc domain fused thereto and 1 ml of OptiPRO^{™} medium was added to a mixture of 80 µl of ExpiFectamine^{™} CHO Reagent (Gibco, 100033021) and 920 µl of OptiPRO^{™} medium, the reaction was conducted at room temperature for 5 minutes, and then the reaction mixture was added to the cultured cells. The cells were cultured at 125 rpm for 20 hours in a shaking incubator maintained at 8% CO₂. Thereafter, 150 µl of ExpiFectamine TM CHO enhancer(Gibco), which enhanced the expression of a single domain antibody having a human IgG Fc domain fused thereto, and 6 ml of ExpiCHO Feed (Gibco) were added, and culture was performed for 5 days at 125 rpm in a 32°C shaking incubator maintained at 5% CO₂. The cultured cells were centrifuged at 4°C and 4,000 rpm for 30 minutes, and the supernatant was filtered using a 0.2 um syringe filter. Thereafter, the supernatant was loaded onto a HiTrap protein G HP column (GE Healthcare), washing with PBS was performed, and then the single domain antibody having a human IgG Fc domain fused thereto was eluted from the column using IgG elution buffer (Thermo). The eluted sample was neutralized by adding 1 M Tris-HCl (pH 9.0) and stored at 4°C until use.

### <Example 7> Evaluation of binding capacity of single domain antibody having human IgG Fc domain fused thereto to immune antigen using FACS

Binding of the anti-CD47 HCAbs (CD47 Nb#01-IgG1 and CD47 Nb#02-IgG1) purified in <Example 6> to an immune antigen was examined by FACS.

Specifically, the Expi-CHO_CD47 cell line (Expi-CHO cells that have overexpressed the CD47 antigen) was washed with DPBS and centrifuged at room temperature and 1,200 rpm for 3 minutes. The supernatant was removed, the cells were resuspended in 2% skim milk, and blocking was performed at 4°C for 30 minutes. The cells were centrifuged at room temperature and 1,200 rpm for 3 minutes to remove the supernatant, and then washed with DPBS two times. Thereafter, the cells were dispensed into each well at 3 × 10⁵ cells/100 µl/well, and treated with the anti-CD47 HCAbs (CD47 Nb#01-IgG1 and CD47 Nb#02-IgG1) at each concentration, respectively, and an isotype control antibody was used as a negative control. The cells were reacted at 4°C for 1 hour and then washed with DPBS two times. Thereafter, the cells were treated with an antibody (anti-human IgG Fc APC antibody (Biolegend)) specifically binding to a human Fc domain, and the reaction was conducted at 4°C for 30 minutes while light was blocked. The cells were washed with DPBS two times, resuspended in 100 µl of DPBS, and subjected to FACS analysis using an Accuri C6 (BD) instrument.

### <Example 8> Evaluation of inhibitory ability of single domain antibody having human IgG Fc domain fused thereto on immune-antigen interaction using FACS

The inhibitory ability of the anti-CD47 HCAbs (CD47 Nb#01-IgG1 and CD47 Nb#02-IgG1) purified in <Example 6> on the CD47/SIRPalpha interaction was evaluated.

Specifically, for the evaluation of anti-CD47 HCAbs (CD47 Nb#01-IgG1 and CD47 Nb#02-IgG1) on the CD47/SIRPalpha interaction, Expi-CHO_CD47 cells (Expi-CHO cells that constantly express the CD47 antigen) were dispensed into a 96-well microplate at 2 × 10⁵ cells per well and treated with 10 µg/ml of human SIRPalpha-His protein. Thereafter, the cells were treated with the anti-CD47 HCAbs (CD47 Nb#01-IgG1 and CD47 Nb#02-IgG1) at each concentration, respectively, and the negative control was treated with an isotype control antibody. The cells were reacted at 4°C for 1 hour and washed with DPBS three times, and then an antibody (Goat anti-His PE) that specifically bound to the His antigen was added, and the reaction was conducted at 4°C for 30 minutes while light was blocked. The cells were washed with DPBS three times and then resuspended in 100 µl of DPBS, and the amount of SIRPalpha-His protein remaining in the Expi-CHO_CD47 cells (Expi-CHO cells that constantly express the CD47 antigen) was measured using an Accuri C6 (BD) instrument to evaluate the inhibitory ability of anti-CD47 HCAbs (CD47 Nb#01-IgG1 and CD47 Nb#02-IgG1) on the CD47/SIRPalpha interaction.

### <Example 9> Evaluation of affinity of single domain antibody having human IgG Fc domain fused thereto for immune antigen

The affinity (Kd) of the anti-CD47 HCAbs (CD47 Nb#01-IgG1 and CD47 Nb#02-IgG1) purified in <Example 6> for an immune antigen protein was measured using an Octet RED 96e (ForteBio) instrument.

Specifically, anti-human Fc-coated biosensor tips (Fortebio) were bound to saturation at a level of 1.5 nm in 96-well microplates (Greiner) to which 5 µg/ml of anti-CD47 HCAbs (CD47 Nb#01-IgG1 and CD47 Nb#02-IgG1) were dispensed, respectively. The CD47 antigen was diluted to 10 to 400 nM step-by-step by 2-fold using 1X kinetic buffer (ForteBio), and the reaction was conducted while stirring was performed at 30°C and 1,000 rpm. The binding and dissociation reactions of the samples were analyzed for 200 and 400 seconds, respectively. The resulting data was analyzed using a 1:1 interaction model (global fitting) method.

### <Example 10> Evaluation of in vitro efficacy and safety of anti-CD47 HCAb (CD47 Nb-IgG4)

The in vitro efficacy of the anti-CD47 HCAb (CD47 Nb-IgG4) purified in <Example 6> was evaluated by the degree of activation of phagocytosis of macrophages by CD47 antigen-specific binding, by human RBC binding, and by hemagglutination.

Specifically, 5 × 10⁶ THP-1 cells were dispensed into 100 pie dishes and cultured for 24 hours in an incubator maintained at 5% CO₂. Thereafter, the cells were reacted with 40 nM PMA for 24 hours, then the medium was removed, and the cells were stained with 1 µM deep red dye. After staining, the medium was replaced, and the cells were rested for 48 hours. THP-1 cells detached with trypsin were mixed with prey cells (Raji cells) stained with 3 µM CFSE at a ratio of 8:1, and then the mixed cells were treated with the anti-CD47 HCAb (CD47 Nb-IgG4) at each concentration and co-cultured for 4 hours. Thereafter, the degree of phagocytosis was evaluated using FACS.

In order to examine the binding capacity of the anti-CD47 HCAb (CD47 Nb-IgG4) to human RBC (red blood cell), RBC was washed with DPBS seven times and then diluted with DPBS to 120 (v/v). After 50 µl of 2× anti-CD47 HCAb (CD47 Nb-IgG4) was dispensed into a 96 well V bottom plate, 50 µl of 12% (v / v) RBC was added, and the reaction was conducted at 4°C for 1 hour. The cells were washed with DPBS, treated with an antibody specifically recognizing human IgG4 (anti-human IgG4) as a secondary antibody, and reacted at 4°C for 1 hour. After the cells were washed with DPBS, the degree of anti-CD47 HCAb (CD47 Nb-IgG4) bound to RBC was evaluated by FACS.

In order to evaluate the hemagglutination of anti-CD47 HCAb (CD47 Nb-IgG4), the human RBC was washed with DPBS seven times and then diluted with DPBS to 6% (v/v). After 50 µl of 2× anti-CD47 HCAb (CD47 Nb-IgG4) was dispensed into a 96 well U bottom plate, 50 µl of 6% (v / v) human RBC was added. After reaction at room temperature for 1 hour, hemagglutination by anti-CD47 HCAb (CD47 Nb-IgG4) was evaluated while being visually observed.

### <Example 11> Evaluation of in vivo efficacy of anti-CD47 HCAb (CD47 Nb-IgG4)

As the evaluation of in vivo efficacy of the anti-CD47 HCAb (CD47 Nb-IgG4) purified in <Example 6>, a tumor cell line (B16F10 cells) induced to express human CD47 was injected into C57BL/6 mice and the antitumor effect of the anti-CD47 HCAb (CD47 Nb-IgG4) was evaluated.

Specifically, 8 × 10⁵ cells/100 µl of B16F10_CD47 cell line (B16F10 cells induced to overexpress human CD47) was injected into 6 to 8 week old C57BL/6 female mice. Tumor formation was induced until the tumor size reached 3 × 3 mm as a width × length size. Thereafter, 10 mpk of anti-CD47 HCAb (CD47 Nb-IgG4) was intraperitoneally administered seven times every 2 days, and the tumor size was measured. The in vivo efficacy of the anti-CD47 HCAb (CD47 Nb-IgG4) was evaluated by measuring the tumor size for 1 week every 2 days after the last intraperitoneal administration.

### <Experimental Example 1> Construction of anti-CD47 HCAbs (CD47 Nb#01-IgG1 and CD47 Nb#02-IgG1)

Using human CD47 protein as an immune antigen, single domain antibody clones specific to the CD47 protein were selected and subjected to sequencing in the same manner as described in <Example 5>. The amino acid sequences of the selected anti-CD47 sdAbs (CD47 Nb#01 and CD47 Nb#02) are presented in Table 5 and Table 6 below.

In addition, using the selected anti-CD47 sdAbs (CD47 Nb#01 and CD47 Nb#02), single domain antibodies specific to CD47 containing a human IgG1 Fc domain were expressed and purified in the same manner as described in <Example 6>, and the purified single domain antibodies were designated anti-CD47 HCAb (CD47 Nb#01-IgG1) and anti-CD47 HCAb (CD47 Nb#02-IgG1), respectively. The amino acid sequences of anti-CD47 HCAbs (CD47 Nb#01-IgG1 and CD47 Nb#02-IgG1) containing a human IgG1 Fc domain are presented in Table 7 below.

In addition, using the selected clone anti-CD47 sdAb (CD47 Nb#01), a single domain antibody specific to CD47 containing human IgG4 Fc was expressed and purified in the same manner as described in <Example 6>, and the purified single domain antibody was designated anti-CD47 HCAb (CD47 Nb-IgG4). The amino acid sequence of anti-CD47 HCAb (CD47 Nb-IgG4) containing a human IgG4 Fc domain is presented in Table 8 below.

**[Table 5]**

| Single domain antibody | Single domain antibody sequence | SEQ ID NO: |
|---|---|---|
| Anti-CD47 sdAb (CD47 Nb#01) | QVQLVESGGGLVQPGGSLNLSCAVSGLTFFRKYAVGWARQVPGKGLERVS RIYSDRRIEYLDAVKGRFTISRDNAKNTVYLQMNSLKTEDTAVYYCATGL RVGLTGWDEYDYWGQGTQVTVSS | 1 |
| Anti-CD47 sdAb (CD47 Nb#02) | QVQLVESGGGLVQPGGSLRLSCTASGSTLNYYIISWFRQTPGKEREGVSC IGSSGAGTNYAASVKGRFTISRDDAQNTVYLFMNDLKPEDTAVYYCAARP FSFSPFCTFAGMKDWGKGTLVTVSS | 2 |

**[Table 6]**

| Single domain antibody | CDR1 | SEQ ID NO: | CDR2 | SEQ ID NO: | CDR3 | SEQ ID NO: |
|---|---|---|---|---|---|---|
| Anti-CD47 sdAb (CD47 Nb#01) | GLTFFRKYA | 3 | IYSDRRI | 4 | ATGLRVGLTGWDEYDY | 5 |
| Anti-CD47 sdAb (CD47 Nb#02) | GSTLNYYI | 6 | IGSSGAGT | 7 | AARPFSFSPFCTFAGMKD | 8 |

**[Table 7]**

| Single domain antibody | Single domain antibody sequence + Human IgG1 Fc sequence | SEQ ID NO: |
|---|---|---|
| Anti-CD47 HCAb (CD47 Nb#01-IgG1) | QVQLVESGGGLVQPGGSLNLSCAVSGLTFFRKYAVGWARQVPGKGLERVS RIYSDRRIEYLDAVKGRFTISRDNAKNTVYLQMNSLKTEDTAVYYCATGL RVGLTGWDEYDYWGQGTQVTVSSGPGGPEPKSSDKTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYT QKSLSLSPGK | 9 |
| Anti-CD47 HCAb (CD47 Nb#02-IgG1) | QVQLVESGGGLVQPGGSLRLSCTASGSTLNYYIISWFRQTPGKEREGVSC IGSSGAGTNYAASVKGRFTISRDDAQNTVYLFMNDLKPEDTAVYYCAARP FSFSPFCTFAGMKDWGKGTLVTVSSGPGGPEPKSSDKTHTCPPCPAPELL GGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKT ISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHNH YTQKSLSLSPGK | 10 |

**[Table 8]**

| Single domain antibody | Single domain antibody sequence + Human IgG4 Fc sequence | SEQ ID NO: |
|---|---|---|
| Anti-CD47 HCAb (CD47 Nb-IgG4) | QVQLVESGGGLVQPGGSLNLSCAVSGLTFFRKYAVGWARQVPGKGLERVS RIYSDRRIEYLDAVKGRFTISRDNAKNTVYLQMNSLKTEDTAVYYCATGL RVGLTGWDEYDYWGQGTQVTVSSGPGGPESKYGPPCPPCPAPEFLGGPSV FLFPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKAK GQPREPQVYTLPPSQEEMTKNQVSLTCLVKGFYPSDIAVEWESNGQPENN YKTTPPVLDSDGSFFLYSRLTVDKSRWQEGNVFSCSVMHEALHNHYTQKS LSLSPGK | 19 |

### <Experimental Example 2> Evaluation of antigen-binding capacity and inhibitory ability on CD47/SIRPalpha interaction of anti-CD47 HCAbs (CD47 Nb#01-IgG1 and CD47 Nb#02-IgG1) and anti-CD47 HCAb (CD47 Nb-IgG4)

The antigen-binding capacity (FIGS. 1A and 2A) and inhibitory ability on the CD47/SIRPalpha interaction (FIGS. 1B and 2B) of the anti-CD47 HCAbs (CD47 Nb#01-IgG1 and CD47 Nb#02-IgG1) and anti-CD47 HCAb (CD47 Nb-IgG4) purified in <Experimental Example 1> were evaluated using FACS in the same manner as described in <Example 7> and <Example 8>.

As a result, as illustrated in FIG. 1A, the anti-CD47 HCAbs (CD47 Nb#01-IgG1 and CD47 Nb#02-IgG1) have been confirmed to have antigen-binding capacities of 9.34 and 14.64 nM (EC50), respectively, in Expi-CHO_CD47 cells (Expi-CHO cells that constantly express the CD47 antigen).

In addition, as illustrated in FIG. 1B, the anti-CD47 HCAbs (CD47 Nb#01-IgG1 and CD47 Nb#02-IgG1) have been confirmed to have inhibitory abilities of on the CD47/SIRPalpha interaction of 43.71 and 75.25 nM (IC50), respectively.

In addition, as illustrated in FIGS. 2A and 2B, the anti-CD47 HCAb (CD47 Nb-IgG4) has been confirmed to have a binding capacity of 3.78 nM (EC50) in Expi-CHO_CD47 cells (Expi-CHO cells that constantly express the CD47 antigen) and an inhibitory ability on the CD47/SIRPalpha interaction of 7.28 nM (IC50).

### <Experimental Example 3> Evaluation of affinity of anti-CD47 HCAbs (CD47 Nb#01-IgG1 and CD47 Nb#02-IgG1) and anti-CD47 HCAb (CD47 Nb-IgG4) for immune antigen

The affinity of each of the anti-CD47 HCAbs (CD47 Nb#01-IgG1 and CD47 Nb#02-IgG1) and anti-CD47 HCAb (CD47 Nb-IgG4) purified in <Experimental Example 1> for the CD47 protein was evaluated in the same manner as described in <Example 9>.

As a result, as presented in Table 9, the anti-CD47 HCAbs (CD47 Nb#01-IgG1 and CD47 Nb#02-IgG1) have been confirmed to have antigen affinities for the CD47 protein of 3.84 and 5.07 nM, respectively, and the anti-CD47 HCAb (CD47 Nb-IgG4) has been confirmed to have an antigenic affinity for the CD47 protein of 2.78 nM.

**[Table 9]**

| Single domain antibody | K_{d} (M) | Kₒₙ (1/Ms) | K_{dis} (1/S) |
|---|---|---|---|
| Anti-CD47 HCAb (CD47 Nb#01-IgG1) | 3.84E-09 | 1.61E+05 | 6.21E-04 |
| Anti-CD47 HCAb (CD47 Nb#02-IgG1) | 5.07E-09 | 1.42E+05 | 7.21E-04 |
| Anti-CD47 HCAb (CD47 Nb-IgG4) | 2.78E-09 | 2.75E+05 | 7.65E-04 |

### <Experimental Example 4> Evaluation of in vitro efficacy and safety of anti-CD47 HCAb (CD47 Nb-IgG4)

In vitro efficacy and safety were evaluated using the anti-CD47 HCAb (CD47 Nb-IgG4) purified in <Experimental Example 1>.

### <4-1> Evaluation of phagocytosis of anti-CD47 HCAb (CD47 Nb-IgG4)

To examine the in vitro efficacy of the anti-CD47 HCAb (CD47 Nb-IgG4) against the CD47 antigen, THP-1 cells were converted into macrophages by PMA in the same manner as described in <Example 10>. Thereafter, the degree of phagocytosis by macrophages through selective binding to prey cells (Raji cells) expressing the CD47 antigen was evaluated.

As a result, as illustrated in FIG. 3, it has been confirmed that the anti-CD47 HCAb (CD47 Nb-IgG4) interferes with THP-1 cells and prey cells (Raji cells) expressing the CD47 antigen to have a binding capacity of 0.14 nM (EC50) to the prey cells expressing CD47.

### <4-2> Evaluation of human RBC binding capacity of anti-CD47 HCAb (CD47 Nb-IgG4)

Since human RBCs express CD47, antibodies that specifically bind to the CD47 antigen may cause side effects such as anemia through binding to human RBCs. Therefore, the safety of the antibody was evaluated by examining the binding capacity of the anti-CD47 HCAb (CD47 Nb-IgG4) to human RBC. The binding capacity to human RBC was evaluated using FACS in the same manner as described in <Example 10>.

As a result, as illustrated in FIG. 4, binding of the anti-CD47 HCAb (CD47 Nb-IgG4) to RBC has not been confirmed even at 3 µM, the maximum concentration in the experiment, whereas binding of the CD47 monoclonal antibody (Competitor) used as a positive control to human RBC has been confirmed at 2.93 nM or more.

### <4-3> Evaluation of hemagglutination of anti-CD47 HCAb (CD47 Nb-IgG4)

Binding of the CD47 antigen-specific antibodies to human RBC induces hemagglutination. Therefore, the safety of antibody was examined by evaluating binding of the anti-CD47 HCAb (CD47 Nb-IgG4) to red blood cells for hemagglutination in the same manner as described in <Example 10>.

As a result, as illustrated in FIG. 5, hemagglutination has not been confirmed at concentrations less than 187.5 nM in the anti-CD47 HCAb (CD47 Nb-IgG4), whereas hemagglutination has been confirmed at 11.72 nM or more in the CD47 monoclonal antibody (Competitor) used as a positive control.

### <Experimental Example 5> Evaluation of in vivo efficacy of anti-CD47 HCAb (CD47 Nb-IgG4)

The evaluation of in vivo efficacy of the anti-CD47 HCAb (CD47 Nb-IgG4) purified in <Experimental Example 1> was performed using a mouse tumor model.

Specifically, in the same manner as described in <Example 11>, the anti-CD47 HCAb (CD47 Nb-IgG4) was intraperitoneally administered to a mouse model in which tumors were formed by injecting tumor cells (B16F10 cells) expressing the CD47 antigen, and the antitumor effect was observed.

As a result, as illustrated in FIG. 6, it has been confirmed that the anti-CD47 HCAb (CD47 Nb-IgG4) has an antitumor effect of about 55.4% compared to that of the negative control (Isotype group).

### [Industrial Applicability]

The single domain antibody according to the present invention exhibits excellent affinity for CD47, an immune checkpoint protein, and excellent antitumor effect, and can be usefully used as an immune checkpoint inhibitor in cancer immunotherapy.

## Claims

1. An antibody comprising a single domain antibody (sdAb) that specifically binds to CD47; or an antigen-binding fragment thereof,
wherein the sdAb includes CDR1 consisting of an amino acid sequence represented by any one of SEQ ID NOs: 3 and 6; CDR2 consisting of an amino acid sequence represented by any one of SEQ ID NOs: 4 and 7; and CDR3 consisting of an amino acid sequence represented by any one of SEQ ID NOs: 5 and 8.

2. The antibody or an antigen-binding fragment thereof according to claim 1, wherein the sdAb includes any one of the following:
(1) CDR1 consisting of an amino acid sequence represented by SEQ ID NO: 3;
CDR2 consisting of an amino acid sequence represented by SEQ ID NO: 4; and
CDR3 consisting of an amino acid sequence represented by SEQ ID NO: 5; or
(2) CDR1 consisting of an amino acid sequence represented by SEQ ID NO: 6;
CDR2 consisting of an amino acid sequence represented by SEQ ID NO: 7; and
CDR3 consisting of an amino acid sequence represented by SEQ ID NO: 8.

3. The antibody or an antigen-binding fragment thereof according to claim 1, wherein the sdAb includes the following VHH domain,
(1) FR1 consisting of an amino acid sequence represented by any one of SEQ ID NOs: 11 and 15;
(2) FR2 consisting of an amino acid sequence represented by any one of SEQ ID NOs: 12 and 16;
(3) FR3 consisting of an amino acid sequence represented by any one of SEQ ID NOs: 13 and 17; and
(4) FR4 consisting of an amino acid sequence represented by any one of SEQ ID NOs: 14 and 18.

4. The antibody or an antigen-binding fragment thereof according to claim 3, wherein the sdAb consists of an amino acid sequence represented by any one of SEQ ID NOs: 1 and 2,

5. The antibody or an antigen-binding fragment thereof according to any one of claims 1 to 4, comprising at least one or more amino acid substitutions.

6. The antibody or an antigen-binding fragment thereof according to claim 5, wherein the at least one or more amino acid substitutions are conservative substitutions.

7. The antibody or an antigen-binding fragment thereof according to claim 6, wherein the at least one or more amino acid substitutions are a substitution of an amino acid with a non-genetically encoded amino acid or a synthetic amino acid.

8. The antibody or an antigen-binding fragment thereof according to any one of claims 1 to 4, which is a heavy chain-only antibody (HCAb) obtained by fusing the sdAb to an Fc fragment.

9. The antibody or an antigen-binding fragment thereof according to claim 8, wherein the HCAb is monomeric or multimeric.

10. The antibody or an antigen-binding fragment thereof according to claim 8, wherein the Fc fragment is human IgG1, IgG2, IgG3 or IgG4.

11. The antibody or an antigen-binding fragment thereof according to claim 8, wherein the sdAb is fused to an Fc fragment via a peptide linker.

12. The antibody or an antigen-binding fragment thereof according to claim 8, wherein the HCAb consists of an amino acid sequence represented by any one of SEQ ID NOs: 9, 10 and 19.

13. The antibody or an antigen-binding fragment thereof according to claim 12, comprising at least one or more amino acid substitutions.

14. The antibody or an antigen-binding fragment thereof according to claim 13, wherein the at least one or more amino acid substitutions are conservative substitutions.

15. The antibody or an antigen-binding fragment thereof according to claim 14, wherein the at least one or more amino acid substitutions are a substitution of an amino acid with a non-genetically encoded amino acid or a synthetic amino acid.

16. The antibody or an antigen-binding fragment thereof according to claim 1, comprising (a) a first antigen-binding moiety including the sdAb; and (b) a second antigen-binding moiety that specifically binds to a second epitope.

17. The antibody or an antigen-binding fragment thereof according to claim 16, wherein the second antigen-binding moiety is a full-length antibody, Fab, Fab', (Fab')2, Fv, single chain Fv (scFv), scFv-scFv, minibody, diabody or second sdAb.

18. The antibody or an antigen-binding fragment thereof according to claim 16, wherein the first antigen-binding moiety and the second antigen-binding moiety are fused to each other via a peptide linker.

19. The antibody or an antigen-binding fragment thereof according to claim 1, which is conjugated to an immunomodulator, cytokine, cytotoxic agent, chemotherapeutic agent, diagnostic agent, antiviral agent, antimicrobial agent or drug.

20. An antibody conjugate comprising the antibody or an antigen-binding fragment thereof according to claim 1, which is conjugated to an immunomodulator, cytokine, cytotoxic agent, chemotherapeutic agent, diagnostic agent, antiviral agent, antimicrobial agent or drug.

21. A nucleic acid molecule encoding the antibody or an antigen-binding fragment thereof according to claim 1.

22. An expression vector comprising the nucleic acid molecule according to claim 21.

23. A host cell transformed with the expression vector according to claim 22.

24. A method for producing an antibody or an antigen-binding fragment thereof, the method comprising:
(a) culturing the host cell according to claim 23 under conditions that allow expression of the antibody; and
(b) recovering the expressed antibody or an antigen-binding fragment thereof.

25. A pharmaceutical composition for prevention or treatment of cancer, comprising the antibody or an antigen-binding fragment thereof according to claim 1 or the antibody conjugate according to claim 20 as an active ingredient.

26. The pharmaceutical composition according to claim 25, wherein the cancer is selected from the group consisting of melanoma, lung cancer, liver cancer, glioblastoma, ovarian cancer, colorectal cancer, head and neck cancer, bladder cancer, renal cell cancer, stomach cancer, breast cancer, metastatic cancer, prostate cancer, pancreatic cancer, non-Hodgkin's lymphoma, Hodgkin's lymphoma, multiple myeloma, leukemia, lymphoma, myelodysplastic syndrome, acute lymphoblastic leukemia, acute myelogenous leukemia, chronic lymphocytic leukemia, chronic myelogenous leukemia, solitary myeloma and aplastic anemia.

27. The pharmaceutical composition according to claim 25, further comprising a pharmaceutically acceptable carrier.

28. A method for preventing or treating cancer, the method comprising administering the antibody or an antigen-binding fragment thereof according to claim 1 or the antibody conjugate according to claim 20 to an individual in a pharmaceutically effective amount.

29. A use of the antibody or an antigen-binding fragment thereof according to claim 1 or the antibody conjugate according to claim 20 for prevention or treatment of cancer.

30. A method for detecting CD47 or determining an amount of CD47 in a sample, the method comprising:
(i) bringing the sample into contact with the antibody or an antigen-binding fragment according to claim 1; and
(ii) detecting formation of a complex between the antibody or an antigen-binding fragment thereof and CD47 or determining an amount of the complex.
